# EUROPÄISCHE PATENTSCHRIFT

(11) **EP 4 146 339 B1**
(45) Veröffentlichungstag und Bekanntmachung des Hinweises auf die Patenterteilung: **10.04.2024**
(21) Anmeldenummer: 20772006.1
(22) Anmeldetag: 07.09.2020
(51) Int. Cl.: A61P 3/00, C07C 67/08, C07C 69/675, C07C 69/70, C07C 69/704, A61K 31/225

(54) **OXOBUTANOL-ESTER VON POLYMEREN CARBONSÄUREN UND DEREN HERSTELLUNG**
OXOBUTANOL ESTERS OF POLYMERIC CARBOXYLIC ACIDS AND THEIR PREPARATION
ESTERS D'OXOBUTANOL D'ACIDES CARBOXYLIQUES POLYMÈRES ET LEUR PRÉPARATION

(30) Priorität: 13.07.2020 WO PCT/EP2020/069709
(43) Veröffentlichungstag der Anmeldung: 15.03.2023
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Von Rohr Patentanwälte Partnerschaft mbB
(86) Internationale Anmeldenummer: PCT/EP2020/074891
(87) Internationale Veröffentlichungsnummer: WO 2022/012766

(56) Entgegenhaltungen:
- US-A1- 2019 177 673

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Oxobutanol-Estern von polymeren Carbonsäuren sowie die auf diese Weise erhältlichen bzw. hergestellten Reaktionsprodukte (d. h. Oxobutanol-Ester von polymeren Carbonsäuren) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Oxobutanol-Ester von polymeren Carbonsäuren) umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel (*Functional Food*)*, Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. Oxobutanol-Ester von polymeren Carbonsäuren) umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die US 2019/177673 betrifft ein Getränk, welches D-Ethyl-3-Hydroxybutyrat, D-1,3-Butandiol, D-β-Hydroxybutyratsalze, D-β-Hydroxybutyrat/D-1,3-Butandiol-monoester, D-Ethylhydroxybutyrat, Hydroxybutyrat und/oder D-Hydroxybuttersäure mit mindestens 5 Vol.-% enthält, wobei der Anteil an D-Ethyl-3-Hydroxybutyrat, D-1,3-Butandiol, D-β-Hydroxybutyratsalze, D-β-Hydroxybutyrat/D-1,3-Butandiol-monoester, D-Ethylhydroxybutyrat, Hydroxybutyrat und/oder D-Hydroxybuttersäure zwischen 5 und 19 % liegt, oder aber höher als 19 % sein kann, wobei das Getränk durch das Hinzufügen von Geschmacksstoffen aus Hopfen und/oder anderen Zutaten verbessert werden kann.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Oxobutanol-Ester von polymeren Carbonsäuren einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Oxobutanol-Estern von polymeren Carbonsäuren gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - ein nach dem erfindungsgemäßen Verfahren erhältliches Reaktionsprodukt gemäß dem diesbezüglichen unabhängigen Anspruch (Anspruch 8) bzw. einen Oxobutanol-Ester von einer polymeren Carbonsäure gemäß den diesbezüglichen Ansprüchen (Ansprüche 9 bis 11) bzw. ein diesbezüglich erhältliches Gemisch von mindestens zwei Oxobutanol-Estern von polymeren Carbonsäuren, gemäß dem diesbezüglichen Anspruch (Anspruch 12); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 13); weitere, insbesondere besondere und/oder vorteilhafte Ausgestaltungen dieses Erfindungsaspekts sind Gegenstand des diesbezüglichen Unteranspruchs.

Darüber hinaus betrifft die vorliegende Erfindung - gemäß einem **vierten** Aspekt der vorliegenden Erfindung - ein erfindungsgemäßes Reaktionsprodukt bzw. einen erfindungsgemäßen Oxobutanol-Ester von einer polymeren Carbonsäure bzw. ein erfindungsgemäßes Gemisch von mindestens zwei Oxobutanol-Estern von polymeren Carbonsäuren zur Verwendung bei der prophylaktischen und/oder therapeutischen Behandlung von Erkrankungen des menschlichen oder tierischen Körpers gemäß dem diesbezüglichen unabhängigen Anspruch (Anspruch 15).

Des Weiteren betrifft die vorliegende Erfindung - gemäß einem **fünften** Aspekt der vorliegenden Erfindung - die Verwendung eines erfindungsgemäßen Reaktionsprodukts bzw. eines erfindungsgemäßen Oxobutanol-Esters von einer polymeren Carbonsäure bzw. eines erfindungsgemäßen Gemischs von mindestens zwei Oxobutanol-Estern von polymeren Carbonsäuren zur Herstellung eines Arzneimittels zur prophylaktischen und/oder therapeutischen Behandlung von Erkrankungen des menschlichen oder tierischen Körpers gemäß dem diesbezüglichen unabhängigen Anspruch (Anspruch 16).

Darüber hinaus betrifft die vorliegende Erfindung - gemäß einem **sechsten** Aspekt der vorliegenden Erfindung - die Verwendung eines erfindungsgemäßen Reaktionsprodukts bzw. eines erfindungsgemäßen Oxobutanol-Ester von einer polymeren Carbonsäure bzw. eines erfindungsgemäßen Gemischs von mindestens zwei Oxobutanol-Estern von polymeren Carbonsäuren gemäß dem diesbezüglichen unabhängigen Anspruch (Anspruch 17).

Des Weiteren betrifft die vorliegende Erfindung - gemäß einem **siebten** Aspekt der vorliegenden Erfindung - ein Nahrungsmittel und/oder Lebensmittelerzeugnis gemäß dem diesbezüglichen unabhängigen Anspruch (Anspruch 18).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung von Oxobutanol-Estern von polymeren Carbonsäuren,
wobei mindestens ein Oxobutanol der allgemeinen Formel (I)

   CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer wenigstens drei Carboxylgruppen enthaltenden polymeren, insbesondere polykondensierten oder polymerisierten, Carbonsäure (II), ausgewählt aus der Gruppe von Polyweinsäure, Polyäpfelsäure und Polycitronensäure sowie deren Salzen und partiellen Salzen und deren Estern und partiellen Estern und deren Kombinationen und Mischungen, in einer Veresterungsreaktion und/oder unter Veresterungsbedingungen umgesetzt wird,
so dass als Reaktionsprodukt (III) ein oder mehrere Oxobutanol-Ester der polymeren Carbonsäure (II) erhalten wird/werden.

Gemäß der vorliegenden Erfindung wird somit ein Herstellungsverfahren für Oxobutanol-Ester, insbesondere 3-Hydroxybutanoat-Ester, von polymeren Carbonsäuren bereitgestellt. 3-Hydroxybutanoat kann synonym auch als 3-Hydroxybuttersäureester bzw. 4-Oxo-2-butanol bezeichnet werden.

In diesem Zusammenhang sind polymere Carbonsäuren Polymere, welche auf Carbonsäuren basieren (d. h. also Polymere, welche durch Polykondensation oder Polymerisation von Carbonsäuren gebildet werden). Nicht zu verwechseln sind polymere Carbonsäuren mit Polycarbonsäuren, welche Moleküle mit mehr als zwei Carboxylgruppen darstellen. Insbesondere weisen polymere Carbonsäuren mindestens zwei Wiederholungseinheiten auf, welche von einer monomeren Carbonsäure abgeleitet sind bzw. darauf basieren. In diesem Zusammenhang bedeutet "abgeleitet von", dass die Wiederholungseinheit aus der monomeren Carbonsäure gebildet wird, insbesondere durch (Poly-)Kondensation (z. B. Veresterung) mit weiteren gleichen monomeren Carbonsäuren; d. h. die Wiederholungseinheit ist z. B. der insbesondere zweifach veresterte Rest (Dicarboxylat-Rest) der entsprechenden monomeren Carbonsäure.

Genau genommen wird im Rahmen der vorliegenden Erfindung als Oxobutanol (insbesondere auch als 3-Hydroxybutanoat bezeichnet) der allgemeinen Formel (I) entweder ein (C₁-C₅-Alkyl)-3-hydroxybutanoat (= 3-Hydroxybuttersäure-(C₁-C₅-alkyl)-ester), d. h. ein C₁-C₅-Alkyester der 3-Hydroxybuttersäure, welcher synonym auch als ein 4-Oxo-4-(C₁-C₅-alkoxy)-2-butanol bezeichnet werden kann, oder aber ein Hydroxy-C₃-C₅-alkyl)-3-hydroxybutanoat (= 3-Hydroxybuttersäure-(hydroxy-C₃-C₅-alkyl)-ester), d. h. ein Hydroxy-C₃-C₅-alkyester der 3-Hydroxybuttersäure, welcher synonym auch als ein 4-Oxo-4-(hydroxy-C₃-C₅-alkoxy)-2-butanol bezeichnet werden kann, eingesetzt.

In diesem Zusammenhang können die 3-Hydroxybutanoate durch Veresterung der freien 3-Hydroxybuttersäure mit dem entsprechenden Alkohol (z. B. Monoalkohol oder Diol) hergestellt werden. Im Folgenden ist die Synthese von Hydroxybutyl-3-hydroxybutanoat (d. h. 3-Hydroxybutanoat der allgemeinen Formel (I) mit R¹ = Hydroxybutyl) schematisch dargestellt:

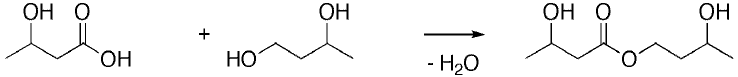

Weiterhin ist im Folgenden die Synthese von Hydroxypentyl-3-hydroxybutanoat (d. h. 3-Hydroxybutanoat der allgemeinen Formel (I) mit R¹ = Hydroxypentyl) schematisch dargestellt:

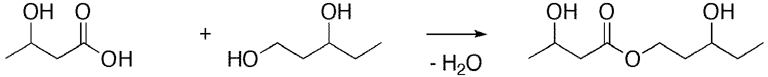

Die Herstellung bzw. Synthese weiterer 3-Hydroxybutanoate verläuft analog.

Im Rahmen des erfindungsgemäßen Verfahrens fungiert also das Edukt bzw. die Ausgangsverbindung Oxobutanol bzw. 3-Hydroxybutanoat der allgemeinen Formel (I) über die Hydroxylgruppe als Veresterungs-Alkohol und reagiert hierüber mit einer Carboxylgruppe der polymeren Carbonsäure (II), sodass als Reaktionsprodukt (III) ein entsprechender Oxobutanol-Ester der polymeren Carbonsäure, insbesondere ein 4-Oxo-4-(C₁-C₅-alkoxy)-2-butanol-Ester bzw. ein 4-Oxo-4-(hydroxy-C₃-C₅-alkoxy)-2-butanol-Ester der polymeren Carbonsäure (II), gebildet wird.

Im Rahmen der vorliegenden Erfindung sind sowohl die eingesetzte polymere Carbonsäure (II) als auch die der polymeren Carbonsäure (II) zugrundeliegende monomere Carbonsäure organische Carbonsäuren (d. h. organische Verbindungen mit einer oder mehreren Carboxylgruppen (-COOH), welche einen aciden Charakter aufweisen).

Durch die Verwendung von polymeren Carbonsäuren kann ein Molekül mit hoher Wirkstoffdichte, insbesondere Dichte an 3-Hydroxybutanoaten bzw. 3-BHB oder entsprechenden Derivaten, bereitgestellt werden.

Darüber hinaus können die Produkte (d. h. Oxobutanol-Ester von polymeren Carbonsäuren) bei Bedarf durch Verwendung entsprechender Edukte und Anpassung der Reaktionsbedingungen in fester Form bereitgestellt werden. Für die Anwendung in oder als Arzneimittel, Medikamente oder Nahrungsmittel- und/oder Lebensmittelerzeugnisse kann eine feste Darreichungsform vorteilhaft sein. Auch weisen Oxobutanol-Ester von polymeren Carbonsäuren - im Vergleich zu entsprechenden Oxobutanol-Estern von monomeren Carbonsäuren - eine andere Löslichkeit auf, welche durch spezifische Auswahl der Edukte und Reaktionsbedingungen beeinflusst werden kann.

Überraschenderweise hat die Anmelderin eine Möglichkeit gefunden 3-Hydroxybuttersäure bzw. deren Derivat (d. h. Ester) in einer organoleptisch kompatiblen Form bereitzustellen, wobei die 3-Hydroxybuttersäure bzw. deren Ester an sich dennoch gut freigesetzt werden kann, insbesondere vom tierischen oder menschlichen Körper.

Darüber hinaus ist es der Anmelderin gelungen, die organoleptisch kompatible Form der 3-Hydroxybuttersäure derart bereitzustellen, dass ein Retardierungseffekt vorliegt, d. h. die 3-Hydroxybuttersäure wird über einen längeren Zeitraum kontinuierlich freigesetzt.

Außerdem sind auch die weiteren Abbau- bzw. Abspaltprodukte (d. h. die Abspaltprodukte, welche neben bzw. zusätzlich zu der 3-Hydroxybuttersäure freigesetzt werden) vom Körper verwertbar, zumindest aber vom Körper verarbeitbar. Insbesondere werden neben 3-BHB Abbau- bzw. Abspaltprodukte freigesetzt, welche Edukte, Produkte oder Zwischenprodukte des Citratzyklus sind oder aber Derivate bzw. Salze sind, welche durch Oxidation eines Edukts, Produkts oder Zwischenprodukts des Citratzyklus gebildet werden. Somit können auch die weiteren Abbau- bzw. Abspaltprodukte, welche bei der Freisetzung von 3-Hydroxybuttersäure gebildet werden, als Energiequelle vom tierischen oder menschlichen Körper genutzt werden.

Durch die Verwendung von polymeren Carbonsäuren liegt eine Vielzahl freier Carboxylgruppen vor, sodass es möglich ist, mehrere Oxobutanole, bzw. 3-Hydroxybutanoate mit der polymeren Carbonsäure reagieren zu lassen (d. h. mehrere Oxobutanole bzw. 3-Hydroxybutanoate werden an eine polymere Carbonsäure addiert), sodass eine hohe Dichte von 3-BHB bzw. deren Ester innerhalb eines Moleküls vorliegt, wodurch eine hohe Wirkstoffdichte erreicht wird.

Wie zuvor ausgeführt, hat die Anmelderin nämlich vollkommen überraschend herausgefunden, dass die auf diese Weise hergestellten Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, effiziente, da physiologisch verträgliche Präkursoren und/oder Metabolite der 3-Hydroxybuttersäure bzw. deren Salzen und Estern darstellen, welche pharmazeutisch bzw. klinisch auch in größeren Mengen zum Einsatz kommen können, da sie physiologisch kompatibel sind.

Die vorgenannten Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen oder Estern dar.

Die Herstellung von Oxobutanol-Estern von polymeren Carbonsäuren, insbesondere 4-Oxo-4-(C₁-C₅-alkoxy)-2-butanol-Estern oder 4-Oxo-4-(hydroxy-C₃-C₅-alkoxy)-2-butanol-Estern von polymeren Carbonsäuren, mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, ohne unerwünschte Nebenreaktionen herstellen lassen, insbesondere einstufig bzw. im Rahmen einer Eintopfsynthese.

Das erfindungsgemäße Verfahren ermöglicht somit erstmalig die Bereitstellung untoxischer Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, aus an sich bekannten, kommerziell verfügbaren und vor allem physiologisch unbedenklichen Komponenten bzw. Edukten (Ausgangsverbindungen) im Rahmen eines wenig komplexen Syntheseverfahrens. Die resultierenden Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, können physiologisch, insbesondere im Magen und/oder im Darm, aufgespalten werden und das Zielmolekül "3-Hydroxybuttersäure" bzw. deren Salze oder Ester als Wirkstoff bzw. Wirkkomponente freisetzen bzw. generieren.

Darüber hinaus weisen die vorgenannten Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren, die Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer von 3-BHB anzureichern, beispielsweise durch Enzymkatalyse oder die gezielte Auswahl der eingesetzten Ausgangsverbindungen (Edukte), um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer von 3-BHB anzureichern.

Weiterhin ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte oder Edukte, welche durch einfache und großtechnisch durchführbar synthetisiert werden können und ermöglicht darüber hinaus insgesamt eine relativ einfache Verfahrensführung auch bei großtechnischer Umsetzung.

Im Gegensatz zu herkömmlichen Herstellungsverfahren des Standes der Technik kommt das erfindungsgemäße Herstellungsverfahren ohne komplexe Edukte aus und verläuft vor allem nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird. Darüber hinaus ist das erfindungsgemäße Verfahren einfach und wirtschaftlich. Insbesondere wird üblicherweise das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*)*;* folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das erfindungsgemäße Herstellungsverfahren führt üblicherweise zu einem Gemisch verschiedener Oxobutanol-Ester von polymeren Carbonsäuren, insbesondere verschiedener 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, d. h. zu einem Gemisch von mindestens zwei, insbesondere drei, voneinander verschiedenen Oxobutanol-Estern von polymeren Carbonsäuren, insbesondere 4-Oxo-4-(C₁-C₅-alkoxy)-2-butanol-Estern oder 4-Oxo-4-(hydroxy-C₃-C₅-alkoxy)-2-butanol-Estern von polymeren Carbonsäuren. Das resultierende Rohreaktionsprodukt bzw. Rohgemisch kann mit an sich bekannten Methoden aufgereinigt werden, insbesondere von gegebenenfalls noch vorhandenen Edukten und/oder gegebenenfalls vorhandenen Nebenprodukten befreit werden, und darüber hinaus - sofern gewünscht - mit ebenfalls an sich bekannten Methoden aufgespalten werden, insbesondere destillativ und/oder chromatographisch (z. B. Fraktionierung in die einzelnen Oxobutanol-Ester von polymeren Carbonsäuren, d. h. Trennung des entsprechenden Monoesters, Diesters etc., oder aber Fraktionierung in Fraktionen mit angereichertem und abgereichertem Anteil einzelner Ester etc.).

Wie zuvor ausgeführt, betrifft die vorliegende Erfindung gemäß dem ersten Aspekt somit ein Verfahren zur Herstellung von Oxobutanol-Estern von polymeren Carbonsäuren,
wobei mindestens ein Oxobutanol der allgemeinen Formel (I)

   CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer wenigstens drei Carboxylgruppen enthaltenden polymeren, insbesondere polykondensierten oder polymerisierten, Carbonsäure (II), ausgewählt aus der Gruppe von Polyweinsäure, Polyäpfelsäure und Polycitronensäure sowie deren Salzen und partiellen Salzen und deren Estern und partiellen Estern und deren Kombinationen und Mischungen, in einer Veresterungsreaktion und/oder unter Veresterungsbedingungen umgesetzt wird,
so dass als Reaktionsprodukt (III) ein oder mehrere Oxobutanol-Ester der polymeren Carbonsäure (II) erhalten wird/werden.

Gemäß einer besonderen Ausführungsform der vorliegenden Erfindung kann die Verbindung der allgemeinen Formel (I) in racemischer Form oder in Form des (R)-Enantiomers eingesetzt werden. Die (R)-Konfiguration bezieht sich auf das chirale Kohlenstoffatom in 3-Position der Verbindung der allgemeinen Formel (I).

Erfindungsgemäß bevorzugt ist es, wenn in der allgemeinen Formel (I) der Rest R¹ Ethyl darstellt.

Mit anderen Worten ist es erfindungsgemäß bevorzugt, dass als Verbindung der allgemeinen Formel (I) Ethyl-3-hydroxybutyrat (synonym auch als 3-Hydroxybuttersäureethylester bzw. 4-Ethoxy-4-oxo-2-butanol bezeichnet) der Formel CH₃ - CH(OH) - CH₂ - C(O)OC₂H₅ eingesetzt wird.

Dies ermöglicht eine besonders effiziente Verfahrensführung und hohe Ausbeuten mit minimierter bzw. unterdrückter Nebenproduktbildung. Zudem ist der 3-Hydroxybuttersäureethylester bzw. 4-Ethoxy-4-oxo-2-butanol auch in großen Mengen kommerziell verfügbar und kann insbesondere großtechnisch z. B. durch Claisen-Kondensation von Ethylacetat gewonnen werden.

Gemäß einer besonderen Ausführungsform der vorliegenden Erfindung kann die polymere Carbonsäure (II) in Form der freien Carbonsäure, in Form eines (partiellen) Salzes der polymeren Carbonsäure oder in Form eines (partiellen) Esters der polymeren Carbonsäure, insbesondere in Form der freien polymeren Carbonsäure, eingesetzt werden.

Im Rahmen der vorliegenden Erfindung kann es vorgesehen sein, dass die polymere Carbonsäure (II) durch Polykondensation oder durch Polymerisation, insbesondere ringöffnende Polymerisation, hergestellt ist.

Bei der Herstellung durch Polykondensation wird z. B. die polymere Carbonsäure durch eine Veresterungsreaktion der Hydroxylgruppe einer Carbonsäure mit der Carboxylgruppe einer weiteren Carbonsäure verestert. In diesem Zusammenhang werden gleiche Carbonsäuren miteinander verestert, d. h. es werden zwei Moleküle der gleichen Carbonsäure, beispielsweise zwei Weinsäuremoleküle oder zwei Citronensäuremoleküle, miteinander verestert. Der Reaktionsverlauf ist am Beispiel der Weinsäure im Folgenden schematisch aufgezeigt:

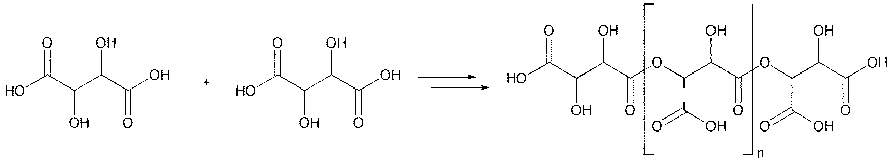

Alternativ kann die polymere Carbonsäure durch ringöffnende Polymerisation des Lactons der Carbonsäure hergestellt werden. Lactone sind heterocyclische Verbindungen, welche eine Esterbindung innerhalb des Rings aufweisen. Der Reaktionsverlauf ist am Beispiel der Weinsäure im Folgenden schematisch aufgezeigt:

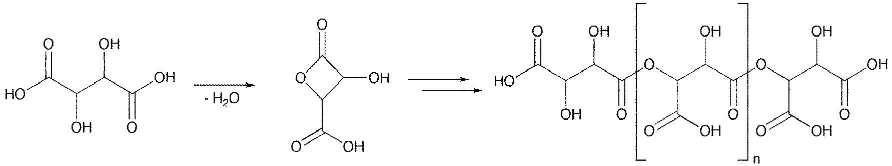

Die entsprechenden Edukte zur Herstellung der polymeren Carbonsäure (d. h. die monomeren Carbonsäuren oder deren entsprechende Lactone) sind käuflich verfügbar und die Polykondensation bzw. ringöffnende Polymerisation ist einfach und großtechnisch durchführbar. Alternativ sind viele polymere Carbonsäuren auch kommerziell verfügbar.

Im Rahmen des erfindungsgemäßen Verfahrens kann es bevorzugt sein, wenn die polymere Carbonsäure (II) ein lebensmittelrechtlich zugelassener Inhaltsstoff, insbesondere Zusatzstoff, ist.

Lebensmittelrechtlich zugelassene Inhaltsstoffe bzw. Zusatzstoffe werden EU-weit in einer Liste für Lebensmittelzusatzstoffe aufgeführt und erhalten in diesem Zusammenhang eine eigene Kennzeichnung (sogenannte E-Nummer). Beispielsweise ist die Metaweinsäure (E353), synonym auch Polyweinsäure, eine lebensmittelrechtlich zugelassene polymere Carbonsäure.

Darüber hinaus kann es auch bevorzugt sein, wenn die polymere Carbonsäure (II) zwei endständige und/oder primäre Carboxylgruppen aufweist. In diesem Zusammenhang liegen die endständigen bzw. primären Carboxylgruppen an den gegenüberliegenden Enden des Moleküls.

Gemäß einer bevorzugten Ausführungsform des erfindungsgemäßen Verfahrens, kann es vorgesehen sein, dass die polymere Carbonsäure (II) mindestens zwei, insbesondere zwei bis zwanzig, vorzugsweise zwei bis zehn, besonders bevorzugt drei bis sieben, von der der polymeren Carbonsäure (II) zugrundeliegenden monomeren Carbonsäure abgeleitete Wiederholungseinheiten enthält.

Die der polymeren Carbonsäure (II) zugrundeliegende monomere Carbonsäure ist die Carbonsäure, welche polykondensiert bzw. polymerisiert wird, um die entsprechende polymere Carbonsäure (II) zu synthetisieren. Beispielsweise ist die Weinsäure die der Polyweinsäure (Metaweinsäure) zugrundeliegende monomere Carbonsäure und im Fall der Polycitronensäure ist die Citronensäure die zugrundeliegende monomere Carbonsäure.

Insbesondere enthalten die Wiederholungseinheiten jeweils mindestens eine freie Carboxylgruppe. Durch die mindestens eine freie Carboxylgruppe in der Wiederholungseinheit können besonders viele Oxobutanole mit der polymeren Carbonsäure verestert werden und somit kann eine hohe Wirkstoffdichte bereitgestellt werden.

Gemäß der Erfindung sind die Wiederholungseinheiten und/oder die der polymeren Carbonsäure (II) zugrundeliegende monomere Carbonsäure abgeleitet von und/oder ausgewählt aus Hydroxydi- und -polycarbonsäuren, vorzugsweise abgeleitet von und/oder ausgewählt aus der Gruppe von Weinsäure, Äpfelsäure und Citronensäure sowie deren Kombinationen und Mischungen, besonders bevorzugt abgeleitet von und/oder ausgewählt von Weinsäure.

In diesem Zusammenhang bedeutet "abgeleitet von", dass die Hydroxydi- und -polycarbonsäuren den Wiederholungseinheiten bzw. der monomere Carbonsäure zugrundeliegen. Im Fall der Wiederholungseinheiten ist diese durch Veresterung der Hydroxydi- und -polycarbonsäuren gebildet und nicht die Hydroxydi- und -polycarbonsäuren an sich bilden die Wiederholungseinheiten, sondern deren (Di-)Carboxylat. Hydroxydi- und -polycarbonsäuren beziehen sich auf organische Stoffe, welche mindestens eine Hydroxylgruppe und mindestens zwei Carboxylgruppen aufweisen.

Diese Carbonsäuren eignen sich besonders für die Umsetzung zu polymeren Carbonsäuren und sind zudem kommerziell erhältlich und darüber hinaus lebensmittelrechtlich zugelassene Zusatzstoffe bzw. Inhaltsstoffe.

Üblicherweise ist die der polymeren Carbonsäure (II) zugrundeliegende monomere Carbonsäure eine natürlich vorkommende Carbonsäure oder deren Derivat, insbesondere Umsetzungsprodukt, insbesondere eine im menschlichen und/oder tierischen Stoffwechsel vorkommende Carbonsäure oder deren Derivat, insbesondere Umsetzungsprodukt.

Polymere Carbonsäuren, welchen Carbonsäuren oder Derivate zugrundeliegen, die Teil des menschlichen und/oder tierischen Stoffwechsels bzw. Edukte bzw. Produkte bzw. Zwischenprodukte eines menschlichen und/oder tierischen Stoffwechsels darstellen, sind besonders kompatibel bei der Verwendung des erhaltenen Reaktionsprodukts in oder als Arzneimittel, Medikament oder Nahrungsmittel- und/oder Lebensmittelerzeugnis. Insbesondere ist es in diesem Zusammenhang vorteilhaft, wenn als die der polymeren Carbonsäure zugrundeliegende monomere Carbonsäuren oder deren Derivate solche verwendet werden, welche im Citratzyklus vorkommen, aus dem Citratzyklus entstehen oder mit dem Citratzyklus im Zusammenhang stehen. Dabei können Derivate beispielsweise Salze oder Ester darstellen, welche durch Oxidation eines Stoffwechselprodukts (beispielsweise aus dem Citratzyklus) erhältlich sind. Durch die Verwendung von polymeren Carbonsäuren, welchen monomere Carbonsäuren oder Derivate zugrundeliegen, die Teil des menschlichen und/oder tierischen Stoffwechsels bzw. Edukte bzw. Produkte bzw. Zwischenprodukte eines menschlichen und/oder tierischen Stoffwechsels darstellen, kann bei der Verwendung des erfindungsgemäßen Reaktionsprodukts dem menschlichen und/oder tierischen Körper eine weitere Energiequelle (zusätzlich zu dem Ketokörper 3-Hydroxybuttersäure bzw. 3-Hydroxybutanoat) zugeführt bzw. bereitgestellt werden.

Insbesondere ist die der polymeren Carbonsäure (II) zugrundeliegende monomere Carbonsäure ein lebensmittelrechtlich zugelassener Inhaltsstoff, insbesondere Zusatzstoff.

Beispielsweise werden die folgenden Carbonsäuren in der Lebensmittelzusatzstoffliste geführt und eignen sich darüber hinaus auch zur Herstellung einer im Rahmen des erfindungsgemäßen Verfahrens verwendbaren polymeren Carbonsäure (II): Weinsäure (E334), Citronensäure (E330) und Äpfelsäure (E296). Diese Säuren sind Teil des Citratzyklus oder durch Oxidation eines Stoffwechselprodukts des Citratzyklus erhältlich. Der Citratzyklus ist ein Kreislauf biochemischer Reaktionen, welcher eine wichtige Rolle im Stoffwechsel (Metabolismus) aerober Zellen von Lebewesen spielt und hauptsächlich dem oxidativen Abbau organischer Stoffe zum Zweck der Energiegewinnung und der Bereitstellung von Zwischenprodukten für Biosynthesen dient. Somit können die Carbonsäuren, welche bei Verwendung des aus dem erfindungsgemäßen Verfahren erhältlichen Reaktionsprodukts (III) durch Abbau gebildet werden, als eine weitere alternative Energiequelle vom Körper verwertet werden.

Gemäß dem erfindungsgemäßen Verfahren, ist die polymere Carbonsäure (II) ausgewählt aus der Gruppe von Polyweinsäure (Metaweinsäure bzw. E 353), Polyäpfelsäure und Polycitronensäure sowie deren (partiellen) Salzen und (partiellen) Estern und deren Kombinationen und Mischungen, besonders bevorzugt aus Polyweinsäure (Metaweinsäure bzw. E 353) sowie deren (partiellen) Salzen und (partiellen) Estern und deren Mischungen, ganz besonders bevorzugt Polyweinsäure (Metaweinsäure bzw. E 353).

In diesem Zusammenhang wird die Polyweinsäure bzw. deren Salze auch als Polytartat, die Polyäpfelsäure bzw. deren Salze auch als Polymalat und die Polycitronensäure bzw. deren Salze auch als Polycitrat bezeichnet.

Gemäß einer der vorliegenden Erfindung ist die polymere Carbonsäure (II) ausgewählt aus Polyweinsäure (Metaweinsäure bzw. E 353) sowie deren (partiellen) Salzen und (partiellen) Estern und deren Mischungen, insbesondere Polyweinsäure (Metaweinsäure bzw. E 353).

Polyweinsäure, synonym auch Metaweinsäure genannt, ist besonders geeignet, da sie ein lebensmittelrechtlich zugelassener Zusatzstoff ist (E-Nummer: E 353) und ist somit besonders geeignet für die Herstellung eines Produkts, welches als Arzneimittel, Medikament oder Nahrungsmittel und/oder Lebensmittelerzeugnis verwendet wird.

Gemäß einer besonderen Ausführungsform der vorliegenden Erfindung kann die polymere Carbonsäure (II) der allgemeinen Formel (IId) 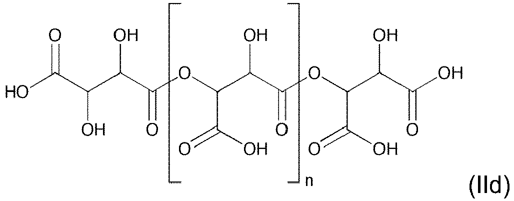 entsprechen, wobei in der allgemeinen Formel (Ild) die Variable n eine (ganze) Zahl ≥ 2, insbesondere im Bereich von zwei bis zwanzig, vorzugsweise im Bereich von zwei bis zehn, besonders bevorzugt im Bereich von drei bis sieben, darstellt.

Gemäß einer besonderen Ausführungsform der vorliegenden Erfindung betrifft die vorliegende Erfindung gemäß diesem Erfindungsaspekt ein Verfahren zur Herstellung von Oxobutanol-Estern von polymeren Carbonsäuren, insbesondere 4-Oxo-4-(C₁-C₅-alkoxy)-2-butanol-Estern oder 4-Oxo-4-(hydroxy-C₃-C₅-alkoxy)-2-butanol-Estern von polymeren Carbonsäuren, insbesondere ein wie zuvor beschriebenes Verfahren,
wobei mindestens ein Oxobutanol, insbesondere mindestens ein 3-Hydroxybutanoat, vorzugsweise mindestens ein 4-Oxo-4-(C₁-C₅-alkoxy)-2-butanol oder 4-Oxo-4-(hydroxy-C₃-C₅-alkoxy)-2-butanol, der allgemeinen Formel (I)

   CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer Carbonsäure (II), ausgewählt aus der Gruppe von Polyweinsäure (Metaweinsäure bzw. E 353), Polyäpfelsäure und Polycitronensäure sowie deren (partiellen) Salzen und (partiellen) Estern und deren Kombinationen und Mischungen, besonders bevorzugt aus Polyweinsäure (Metaweinsäure bzw. E 353) sowie deren (partiellen) Salzen und (partiellen) Estern und deren Mischungen, ganz besonders bevorzugt Polyweinsäure (Metaweinsäure bzw. E 353), in einer Veresterungsreaktion und/oder unter Veresterungsbedingungen umgesetzt wird,
so dass als Reaktionsprodukt (III) ein oder mehrere Oxobutanol-Ester der polymeren Carbonsäure (II), insbesondere ein oder mehrere 4-Oxo-4-(C₁-C₅-alkoxy)-2-butanol-Ester oder 4-Oxo-4-(hydroxy-C₃-C₅-alkoxy)-2-butanol-Ester der polymeren Carbonsäure (II), erhalten werden.

Gemäß einer weiteren besonderen Ausführungsform der vorliegenden Erfindung betrifft die vorliegende Erfindung gemäß diesem Erfindungsaspekt auch ein Verfahren zur Herstellung von Oxobutanol-Estern von polymeren Carbonsäuren, insbesondere 4-Oxo-4-(C₁-C₅-alkoxy)-2-butanol-Estern oder 4-Oxo-4-(hydroxy-C₃-C₅-alkoxy)-2-butanol-Estern von polymeren Carbonsäuren, insbesondere ein wie zuvor beschriebenes Verfahren,
wobei mindestens ein Oxobutanol, insbesondere mindestens ein 3-Hydroxybutanoat, vorzugsweise mindestens ein 4-Oxo-4-(C₁-C₅-alkoxy)-2-butanol oder 4-Oxo-4-(hydroxy-C₃-C₅-alkoxy)-2-butanol, der allgemeinen Formel (I)

   CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer Carbonsäure (II), ausgewählt aus Polyweinsäure (Metaweinsäure bzw. E 353) sowie deren (partiellen) Salzen und (partiellen) Estern und deren Mischungen, insbesondere Polyweinsäure (Metaweinsäure bzw. E 353), in einer Veresterungsreaktion und/oder unter Veresterungsbedingungen umgesetzt wird,
so dass als Reaktionsprodukt (III) ein oder mehrere Oxobutanol-Ester der polymeren Carbonsäure (II), insbesondere ein oder mehrere 4-Oxo-4-(C₁-C₅-alkoxy)-2-butanol-Ester oder 4-Oxo-4-(hydroxy-C₃-C₅-alkoxy)-2-butanol-Ester der polymeren Carbonsäure (II), erhalten werden.

Darüber hinaus betrifft die vorliegende Erfindung gemäß einer besonderen Ausführungsform der vorliegenden Erfindung gemäß diesem Erfindungsaspekt auch ein Verfahren zur Herstellung von Oxobutanol-Estern von polymeren Carbonsäuren, insbesondere 4-Oxo-4-(C₁-C₅-alkoxy)-2-butanol-Estern oder 4-Oxo-4-(hydroxy-C₃-C₅-alkoxy)-2-butanol-Estern von polymeren Carbonsäuren, insbesondere ein wie zuvor beschriebenes Verfahren,
wobei mindestens ein Oxobutanol, insbesondere mindestens ein 3-Hydroxybutanoat, vorzugsweise mindestens ein 4-Oxo-4-(C₁-C₅-alkoxy)-2-butanol oder 4-Oxo-4-(hydroxy-C₃-C₅-alkoxy)-2-butanol, der allgemeinen Formel (I)

   CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer Carbonsäure (II) der allgemeinen Formel (IId) 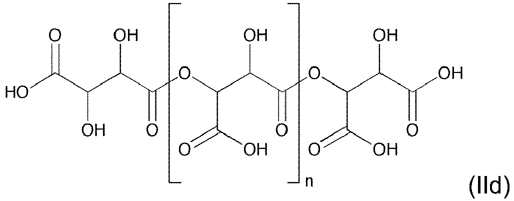
wobei in der allgemeinen Formel (Ild) die Variable n eine (ganze) Zahl ≥ 2, insbesondere im Bereich von zwei bis zwanzig, vorzugsweise im Bereich von zwei bis zehn, besonders bevorzugt im Bereich von drei bis sieben, darstellt in einer Veresterungsreaktion und/oder unter Veresterungsbedingungen umgesetzt wird,
so dass als Reaktionsprodukt (III) ein oder mehrere Oxobutanol-Ester der polymeren Carbonsäure (Ild), insbesondere ein oder mehrere 4-Oxo-4-(C₁-C₅-alkoxy)-2-butanol-Ester oder 4-Oxo-4-(hydroxy-C₃-C₅-alkoxy)-2-butanol-Ester der polymeren Carbonsäure (Ild), erhalten werden.

Gemäß einer besonderen Ausführungsform der vorliegenden Erfindung kann die Umsetzung in Abwesenheit von Lösemitteln und/oder ohne jedwedes Lösemittel durchgeführt werden. Das heißt die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer weiteren besonderen Ausführungsform der vorliegenden Erfindung kann die Umsetzung in Abwesenheit eines Katalysators und/oder ohne jedweden Katalysator durchgeführt werden, oder aber die Umsetzung kann alternativ in Gegenwart eines Katalysators, insbesondere eines Enzyms und/oder eines metallhaltigen und/oder metallbasierten, sauren oder basischen Katalysators durchgeführt werden, wobei der Katalysator nach der Umsetzung rezykliert werden kann.

Im Rahmen des erfindungsgemäßen Verfahrens ist es insbesondere bevorzugt, wenn die Umsetzung in Abwesenheit von Lösemitteln und/oder ohne jedwedes Lösemittel durchgeführt wird und wenn die Umsetzung in Abwesenheit eines Katalysators und/oder ohne jedweden Katalysator durchgeführt wird.

Durch die Abwesenheit von Lösemittel und die Abwesenheit eines Katalysators, werden die Reaktionsprodukte nicht durch nicht vollständig entferntes Lösemittel oder nicht vollständig entferntem Katalysator verunreinigt. Darüber hinaus fallen die energieträchtigen und aufwendigen Entfernungs- bzw. Abtrennungsschritte weg. Überaschenderweise ist das erfindungsgemäße Verfahren gemäß dieser bevorzugten Ausführungsform trotzdem wirtschaftlich und resultiert in hohen Umsätzen ohne signifikante Nebenproduktbildung.

Wie zuvor ausführt, kann gemäß einer besonderen Ausführungsform des erfindungsgemäßen Herstellungsverfahrens die Umsetzung in Abwesenheit eines Katalysators und/oder ohne jedweden Katalysator durchgeführt werden.

Sofern die Umsetzung in Abwesenheit eines Katalysators und/oder ohne jedweden Katalysator durchgeführt wird, ist es bevorzugt, wenn die Umsetzung bei Temperaturen im Bereich von 20 °C bis 160°C, insbesondere im Bereich von 50 °C bis 150°C, vorzugsweise im Bereich von 70 °C bis 140°C, besonders bevorzugt im Bereich von 80 °C bis 135 °C, ganz besonders bevorzugt im Bereich von 100 °C bis 130 °C, durchgeführt wird.

Im Fall der Umsetzung in Abwesenheit eines Katalysators kann der angewendete Druckbereich in weiten Bereichen variieren. Insbesondere kann die Umsetzung in Abwesenheit eines Katalysators und/oder ohne jedweden Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Bei der Umsetzung in Abwesenheit eines Katalysators ist es bevorzugt, wenn die Umsetzung in Gegenwart eines Inertgases, insbesondere in Gegenwart von Helium, Argon oder Stickstoff, bevorzugt in Gegenwart von Stickstoff, durchgeführt wird. Insbesondere können hierdurch unerwünschte Nebenreaktionen, insbesondere aufgrund von Oxidation oder Hydrolyse, verhindert werden.

Alternativ zu dieser besonderen Ausführungsform ist es aber auch möglich, die Umsetzung z. B. in Gegenwart eines Enzyms als Katalysator durchzuführen.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstand sind (Lipolyse).

In diesem Zusammenhang kann sich das als Katalysator eingesetzte Enzym insbesondere ableiten von *Candida antarctica, Mucor miehei* (*Rhizomucor miehei*)*, Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Gemäß einer besonderen Ausführungsform kann das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Im Rahmen der vorliegenden Erfindung ist es bevorzugt, wenn im Fall der Verwendung eines Enzyms als Katalysator das Enzym nach der Umsetzung rezykliert wird.

Sofern die Umsetzung im Rahmen des erfindungsgemäßen Herstellungsverfahrens in Gegenwart eines Enzyms als Katalysator durchgeführt wird, ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Wenn gemäß einer besonderen Ausführungsform der vorliegenden Erfindung die Umsetzung in Gegenwart eines Enzyms als Katalysator durchgeführt wird, kann auch der angewendete Druckbereich in weiten Bereichen variieren. Typischerweise kann die Umsetzung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 0,5 bar, durchgeführt werden.

Gemäß der besonderen Ausführungsform der vorliegenden Erfindung, wonach die Umsetzung in Gegenwart eines Enzyms als Katalysator durchgeführt wird, ist es bevorzugt, wenn die Umsetzung in Gegenwart eines Enzyms in Gegenwart eines Inertgases, insbesondere in Gegenwart von Helium, Argon oder Stickstoff, bevorzugt in Gegenwart von Stickstoff, durchgeführt wird. Wie zuvor bereits im Zusammenhang mit der Umsetzung in Abwesenheit eines Katalysators ausgeführt, können durch die Umsetzung in Gegenwart eines Inertgases unerwünschte Nebenreaktionen, insbesondere aufgrund von Oxidation oder Hydrolyse, verhindert werden.

Gemäß einer weiteren alternativen Ausführungsform der vorliegenden Erfindung kann die Umsetzung z. B. in Gegenwart eins metallhaltigen und/oder metallbasierten, sauren oder basischen Katalysators durchgeführt werden.

Gemäß dieser alternativen Ausführungsform der vorliegenden Erfindung, wonach die Umsetzung in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere eine Lewis-Säure auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl, eingesetzt werden.

Insbesondere ist es auch bei dieser Ausführungsform bevorzugt, wenn der metallhaltige und/oder metallbasierte saure oder basische Katalysator nach der Umsetzung rezykliert wird.

Wenn gemäß der besonderen Ausführungsform der vorliegenden Erfindung die Umsetzung in Gegenwart eines metallhaltigen und/oder metallbasierten, sauren oder basischen Katalysators erfolgt, können die Temperaturen in weiten Bereichen variiert werden. Insbesondere kann die Umsetzung in Gegenwart eines metallhaltigen und/oder metallbasierten, sauren oder basischen Katalysators bei Temperaturen im Bereich von 20 °C bis 160 °C, insbesondere im Bereich von 50 °C bis 150 °C, vorzugsweise im Bereich von 70 °C bis 140 °C, besonders bevorzugt im Bereich von 80 °C bis 135 °C, ganz besonders bevorzugt im Bereich von 100 °C bis 130 °C, durchgeführt werden.

Weiterhin kann auch bei dieser Ausführungsform der Katalysator (d. h. in Gegenwart eines metallhaltigen und/oder metallbasierten, sauren oder basischen Katalysators) in weiten Mengenbereichen variiert werden: So kann der Katalysator in Mengen, bezogen auf die Gesamtmenge der Ausgangsverbindungen (I) und (II), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den vorgenannten Mengen abzuweichen, ohne dass der Rahmen der vorliegenden Erfindung verlassen ist.

Wenn gemäß dieser besonderen Ausführungsform der vorliegenden Erfindung die Umsetzung in Gegenwart eines metallhaltigen und/oder metallbasierten, sauren oder basischen Katalysators erfolgt, kann der Druckbereich gleichermaßen in einem weiten Regime variieren: Insbesondere kann die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Weiterhin kann auch gemäß dieser besonderen Ausführungsform der vorliegenden Erfindung, wonach die Umsetzung in Gegenwart eines metallhaltigen und/oder metallbasierten, sauren oder basischen Katalysators durchgeführt wird, die Umsetzung in Gegenwart eines Inertgases, insbesondere in Gegenwart von Helium, Argon oder Stickstoff, bevorzugt in Gegenwart von Stickstoff, durchgeführt werden. Wie zuvor bereits ausgeführt werden durch die Umsetzung in Gegenwart eines Inertgases unerwünschte Nebenreaktionen, insbesondere aufgrund von Oxidation oder Hydrolyse, verhindert.

Was die Menge an Edukten bzw. Ausgangsverbindungen insgesamt anbelangt, so kann diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn das Oxobutanol der allgemeinen Formel (I), bezogen auf die Carboxylgruppen der polymeren Carbonsäure (II) in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn das Oxobutanol der allgemeinen Formel (I) und die polymere Carbonsäure (II) in einem Molverhältnis von Oxobutanol der allgemeinen Formel (I) / Carboxylgruppen der polymeren Carbonsäure (II) in einem Bereich von 1 :1 bis 10 : 1, insbesondere in einem Bereich von 2: 1 bis 8 : 1, vorzugsweise in einem Bereich von 3: 1 bis 6 : 1, eingesetzt werden.

Im Rahmen des erfindungsgemäßen Verfahrens wird bei der Umsetzung von Oxobutanol der allgemeinen Formel (I) mit einer polymeren Carbonsäure (II) in Form der freien Säure gleichzeitig Wasser gebildet. Insbesondere ist es bevorzugt, wenn das Wasser der Umsetzung entzogen wird, insbesondere kontinuierlich entzogen wird, insbesondere mittels vorzugsweise kontinuierlicher, insbesondere destillativer oder adsorptiver Entfernung.

Alternativ wird im Rahmen des erfindungsgemäßen Verfahrens bei der Umsetzung von Oxobutanol der allgemeinen Formel (I) mit einer polymeren Carbonsäure (II) in Form des Esters pro Mol umgesetzte Estergruppe ein Mol des entsprechenden Alkohols gebildet. Insbesondere ist es bevorzugt, wenn der Alkohol der Umsetzung entzogen wird, insbesondere kontinuierlich entzogen wird, insbesondere mittels vorzugsweise kontinuierlicher, insbesondere destillativer oder adsorptiver Entfernung.

In diesem Zusammenhang bezieht sich die Bereicherung "umgesetzte Estergruppe" auf die Estergruppe, welche im Rahmen des erfindungsgemäßen Verfahren umgeestert wird, d. h. auf die Estergruppe die mit dem Oxobutanol reagiert. In diesem Zusammenhang wird der entsprechende Alkohol abgespalten; d. h. wenn ein Ethylester der polymeren Carbonsäure vorliegt, wird durch die Umesterung mit einem Oxobutanol Ethanol abgespalten.

Durch die kontinuierliche Entfernung des Nebenprodukts (d. h. Wasser bzw. Alkohol) kann insbesondere das chemische Gleichgewicht in Richtung der Produkte verschoben und somit der Umsatz gesteigert werden.

Im Rahmen des erfindungsgemäßen Herstellungsverfahren kann die Zusammensetzung des Reaktionsprodukts, insbesondere das Vorhandensein verschiedener Oxobutanol-Ester von polymeren Carbonsäuren, und deren Anteil im Fall eines Gemischs, mittels der Umsetzungsbedingungen kontrolliert und/oder gesteuert werden, insbesondere durch Auswahl der Umsetzungstemperatur (Reaktionstemperatur) und/oder Auswahl des Umsetzungsdrucks (Reaktionsdrucks) und/oder Abwesenheit oder Vorsehen eines Katalysators und dessen Auswahl in Bezug auf Art und/oder Menge und/oder Auswahl der Mengen der Ausgangsverbindungen (Edukte) und/oder Vorsehen der Entfernung der gegebenenfalls gebildeten Nebenprodukte, insbesondere Wasser und/oder Alkohol.

Somit ist es möglich die genaue Zusammensetzung des Produktes je nach Anwendung maßzuschneidern, insbesondere kann beispielsweise die Anzahl der substituierten Oxobutanol-Reste (3-Hydroxybutanoat-Reste) eingestellt werden, so dass die Dichte an Ketokörpern in Form von Oxobutanolen bzw. 3-Hydroxybutanoaten pro Molekül zielgerichtet eingestellt werden kann.

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann das erhaltene Reaktionsprodukt nach erfolgter Umsetzung fraktioniert werden, insbesondere destillativ fraktioniert werden.

Auch können nichtumgesetzte Ausgangsverbindungen (I) und/oder (II) aus dem Reaktionsprodukt abgetrennt und anschließend rezykliert werden.

Gemäß einer besonderen Ausführungsform des erfindungsgemäßen Herstellungsverfahrens kann insbesondere derart vorgegangen werden, dass im Reaktionsprodukt nach erfolgter Umsetzung noch vorhandene Hydroxylgruppen und/oder Carboxylgruppen zumindest teilweise, vorzugsweise vollständig, funktionalisiert, insbesondere verestert werden.

Insbesondere kann sich der Umsetzung eine teilweise, insbesondere vollständige Funktionalisierung, insbesondere Veresterung, noch vorhandener Hydroxylgruppen und/oder Carboxylgruppen anschließen.

Bei dieser besonderen Ausführungsform des erfindungsgemäßen Verfahrens kann die Funktionalisierung, insbesondere die Veresterung noch vorhandener Hydroxylgruppen und/oder Carboxylgruppen, durch Reaktion mit einem Carbonsäureanhydrid von beispielsweise C₂-C₃₀-Carbonsäuren oder C₂-C₃₀-Fettsäuren in freien Hydroxylgruppen oder C₂-C₃₀-Fettalkoholen im Fall freier Carboxylgruppen erfolgen. Dabei kann es sich um lineare oder verzweigte, gesättigte oder ein- oder mehrfach ungesättigte C₂-C₃₀-Carbonsäureanhydride oder C₂-C₃₀-Fettsäureanhydride oder C₂-C₃₀-Fettalkohole handeln. In diesem Zusammenhang können noch vorhandene Hydroxylgruppen insbesondere mit Carbonsäureanhydriden oder Fettsäuren umgesetzt werden und noch vorhandene Carboxylgruppen können insbesondere mit Fettalkoholen jeweils zu den entsprechenden Estern umgesetzt werden.

Im Rahmen des erfindungsgemäßen Verfahrens können als Reaktionsprodukt (III) ein oder mehrere Oxobutanol-Ester der polymeren Carbonsäure (II), insbesondere ein oder mehrere 4-Oxo-4-(C₁-C₅-alkoxy)-2-butanol-Ester oder 4-Oxo-4-(hydroxy-C₃-C₅-alkoxy)-2-butanol-Ester der polymeren Carbonsäure (II), erhalten werden.

Insbesondere können bei dem erfindungsgemäßen Herstellungsverfahren als Reaktionsprodukt (III) ein oder mehrere Oxobutanol-Ester der polymeren Carbonsäure (II), insbesondere ein oder mehrere 4-Oxo-4-(C₁-C₅-alkoxy)-2-butanol-Ester oder 4-Oxo-4-(hydroxy-C₃-C₅-alkoxy)-2-butanol-Ester der polymeren Carbonsäure (II), wie nachfolgend definiert, erhalten werden.

Gemäß einer besonderen Ausführungsform des erfindungsgemäßen Verfahrens kann als Reaktionsprodukt ein Gemisch von mindestens zwei, insbesondere mindestens drei, voneinander verschiedenen Oxobutanol-Estern der polymeren Carbonsäure (II), insbesondere ein oder mehrere 4-Oxo-4-(C₁-C₅-alkoxy)-2-butanol-Ester oder 4-Oxo-4-(hydroxy-C₃-C₅-alkoxy)-2-butanol-Ester der polymeren Carbonsäure (II), insbesondere wie zuvor und nachfolgend noch definiert, erhalten werden.

Weiterer Gegenstand - gemäß einem **zweiten** Aspekt der vorliegenden Erfindung - ist ein Reaktionsprodukt, insbesondere (chemisches) Produkt oder Produktgemisch, vorzugsweise Oxobutanol-Ester einer polymeren Carbonsäure, insbesondere 4-Oxo-4-(C₁-C₅-alkoxy)-2-butanol-Ester oder 4-Oxo-4-(hydroxy-C₃-C₅-alkoxy)-2-butanol-Ester einer polymeren Carbonsäure, oder ein Gemisch von mehreren Oxobutanol-Estern einer polymeren Carbonsäure, insbesondere Gemisch von mehreren 4-Oxo-4-(C₁-C₅-alkoxy)-2-butanol-Estern oder 4-Oxo-4-(hydroxy-C₃-C₅-alkoxy)-2-butanol-Estern einer polymeren Carbonsäure, welches erhältlich ist gemäß dem zuvor beschriebenen Verfahren.

Gegenstand der vorliegenden Erfindung ist insbesondere auch ein Oxobutanol-Ester einer polymeren Carbonsäure,
wobei die polymere Carbonsäure ausgewählt ist aus der Gruppe von Polyweinsäure, Polyäpfelsäure und Polycitronensäure sowie deren Salzen und partiellen Salzen und Estern und partiellen Estern und deren Kombinationen und Mischungen; und
wobei mindestens eine Carboxylgruppe, insbesondere mindestens eine endständige und/oder primäre Carboxylgruppe, der polymeren Carbonsäure verestert ist mit einem Oxobutanol der allgemeinen Formel (I) CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt.

Gemäß diesem Erfindungsaspektist mindestens eine Carboxylgruppe, insbesondere mindestens eine endständige und/oder primäre Carboxylgruppe, der polymeren Carbonsäure verestert mit einem Oxobutanol, insbesondere mit mindestens einem 3-Hydroxybutanoat, vorzugsweise mit mindestens einem 4-Oxo-4-(C₁-C₅-alkoxy)-2-butanol oder 4-Oxo-4-(hydroxy-C₃-C₅-alkoxy)-2-butanol, der allgemeinen Formel (I)

CH₃-CH(OH)-CH₂-C(O)OR¹ (I)

wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt.

Gemäß einer weiteren besonderen Ausführungsform dieses Erfindungsaspekts kann die polymere Carbonsäure durch Polykondensation oder durch Polymerisation, insbesondere ringöffnende Polymerisation, hergestellt sein.

Gemäß diesem Erfindungsaspekt kann es bevorzugt sein, wenn die polymere Carbonsäure ein lebensmittelrechtlich zugelassener Inhaltsstoff, insbesondere Zusatzstoff, ist. Wie zuvor im Zusammenhang mit dem erfindungsgemäßen Verfahren bereits ausgeführt, sind die erfindungsgemäßen Oxobutanol-Ester von polymeren Carbonsäuren, welche lebensmittelrechtlich zugelassene polymere Carbonsäuren enthalten, besonders geeignet für die Anwendung in oder als Arzneimittel, Medikament, Nahrungsmittel und/oder Lebensmittelerzeugnis.

Weiterhin kann es gemäß diesem Erfindungsaspekt auch bevorzugt sein, wenn die polymere Carbonsäure zwei endständige und/oder primäre Carboxylgruppen aufweist.

Gemäß einer besonderen Ausführungsform dieses Erfindungsaspekts, kann es vorteilhaft sein, wenn die polymere Carbonsäure mindestens zwei, insbesondere zwei bis zwanzig, vorzugsweise zwei bis zehn, besonders bevorzugt drei bis sieben, von der der polymeren Carbonsäure zugrundeliegenden monomeren Carbonsäure abgeleitete Wiederholungseinheiten enthält.

Typischerweise enthalten die Wiederholungseinheiten jeweils mindestens eine freie Carboxylgruppe. Wenn jede Wiederholungseinheit mindestens eine freie Carboxylgruppe enthält, ist es möglich, eine hohe Wirkstoffdichte bereitzustellen, da jede freie Carboxylgruppe durch ein bzw. mit einem Oxobutanol verestert werden kann.

Insbesondere sind die Wiederholungseinheiten und/oder die der polymeren Carbonsäure zugrundeliegende monomere Carbonsäure abgeleitet von und/oder ausgewählt aus Hydroxydi- und -polycarbonsäuren, vorzugsweise abgeleitet von und/oder ausgewählt aus der Gruppe von Weinsäure, Äpfelsäure und Citronensäure sowie deren Kombinationen und Mischungen, besonders bevorzugt abgeleitet von und/oder ausgewählt von Weinsäure.

Wie bereits zuvor ausgeführt, bedeutet "abgeleitet von", dass die Hydroxydi- und -polycarbonsäuren den Wiederholungseinheiten bzw. der monomere Carbonsäure zugrundeliegen. Im Fall der Wiederholungseinheiten ist diese durch Veresterung der Hydroxydi- und -polycarbonsäuren gebildet und nicht die Hydroxydi- und -polycarbonsäuren an sich bilden die Wiederholungseinheiten. Hydroxydi- und -polycarbonsäuren beziehen sich auf organische Stoffe, welche mindestens eine Hydroxylgruppe und mindestens zwei Carboxylgruppen aufweisen.

Vorzugsweise ist die der polymeren Carbonsäure zugrundeliegende monomere Carbonsäure eine natürlich vorkommende Carbonsäure oder deren Derivat, insbesondere Umsetzungsprodukt, insbesondere eine im menschlichen und/oder tierischen Stoffwechsel vorkommende Carbonsäure oder deren Derivat, insbesondere Umsetzungsprodukt.

Wie bereits zuvor ausgeführt, sind polymere Carbonsäuren, welchen Carbonsäuren oder Derivate zugrundeliegen, die Teil des menschlichen und/oder tierischen Stoffwechsels bzw. Edukts bzw. Produkts bzw. Zwischenprodukts eines menschlichen und/oder tierischen Stoffwechsels darstellen, besonders kompatibel bei der Verwendung des erhaltenen Reaktionsprodukts in oder als Arzneimittel, Medikament oder Nahrungsmittel- und/oder Lebensmittelerzeugnis. Insbesondere ist es in diesem Zusammenhang vorteilhaft, wenn als die der polymeren Carbonsäure zugrundeliegende monomere Carbonsäuren oder deren Derivate solche verwendet werden, welche im Citratzyklus vorkommen, aus dem Citratzyklus entstehen oder mit dem Citratzyklus im Zusammenhang stehen. Dabei können Derivate beispielsweise Salze oder Ester darstellen, welche durch Oxidation eines Stoffwechselprodukts (beispielsweise aus dem Citratzyklus) erhältlich sind. Durch die Verwendung von polymeren Carbonsäuren, welchen monomere Carbonsäuren oder Derivate zugrundeliegen, die Teil des menschlichen und/oder tierischen Stoffwechsels bzw. Edukts, Produkts bzw. Zwischenprodukts eines menschlichen und/oder tierischen Stoffwechsels darstellen, kann bei der Verwendung des erfindungsgemäßen Reaktionsprodukts dem menschlichen und/oder tierischen Körper eine weitere Energiequelle (zusätzlich zu dem Ketokörper 3-Hydroxybuttersäure bzw. 3-Hydroxybutanoat) bereitgestellt werden.

Insbesondere ist die der polymeren Carbonsäure zugrundeliegende monomere Carbonsäure ein lebensmittelrechtlich zugelassener Inhaltsstoff, insbesondere Zusatzstoff.

Wie zuvor bereits ausgeführt, werden beispielsweise die folgenden Carbonsäuren in der Lebensmittelzusatzstoffliste geführt und eignen sich darüber hinaus auch zur Herstellung einer im Rahmen des erfindungsgemäßen Verfahrens verwendbaren polymeren Carbonsäure (II): Weinsäure (E334), Citronensäure (E330) und Äpfelsäure (E296). Diese Säuren sind Teil des Citratzyklus oder durch Oxidation eines Stoffwechselprodukts des Citratzyklus erhältlich. Der Citratzyklus ist ein Kreislauf biochemischer Reaktionen, welche eine wichtige Rolle im Stoffwechsel (Metabolismus) aerober Zellen von Lebewesen spielt und hauptsächlich dem oxidativen Abbau organischer Stoffe zum Zweck der Energiegewinnung und der Bereitstellung von Zwischenprodukten für Biosynthesen dient. Somit können die Säuren, welche bei Verwendung des aus dem erfindungsgemäßen Verfahren erhältlichen Reaktionsprodukts (III) durch Abbau gebildet werden, als eine weitere alternative Energiequelle vom Körper verwertet werden.

Gemäß diesem Erfindungsaspekte ist die polymere Carbonsäure ausgewählt aus der Gruppe von Polyweinsäure (Metaweinsäure bzw. E 353), Polyäpfelsäure und Polycitronensäure sowie deren (partiellen) Salzen und (partiellen) Estern und deren Kombinationen und Mischungen, besonders bevorzugt aus Polyweinsäure (Metaweinsäure bzw. E 353) sowie deren (partiellen) Salzen und (partiellen) Estern und deren Mischungen, ganz besonders bevorzugt Polyweinsäure (Metaweinsäure bzw. E 353).

Gemäß diesem Erfindungsaspekte, ist die polymere Carbonsäure ausgewählt aus Polyweinsäure (Metaweinsäure bzw. E 353) sowie deren (partiellen) Salzen und (partiellen) Estern und deren Mischungen, insbesondere Polyweinsäure (Metaweinsäure bzw. E 353).

Weiterhin, gemäß einer besonderen Ausführungsform dieses Erfindungsaspektes, kann die polymere Carbonsäure der allgemeinen Formel (Ild) 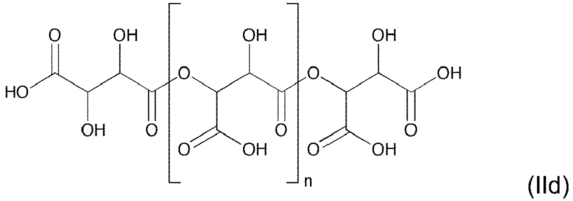 entsprechen, wobei in der allgemeinen Formel (Ild) die Variable n eine (ganze) Zahl ≥ 2, insbesondere im Bereich von zwei bis zwanzig, vorzugsweise im Bereich von zwei bis zehn, besonders bevorzugt im Bereich von drei bis sieben, darstellt.

Darüber hinaus ist auch Gegenstand der vorliegenden Erfindung gemäß einer wiederum weiteren besonderen Ausführungsform ein Oxobutanol-Ester einer polymeren Carbonsäure, insbesondere 4-Oxo-4-(C₁-C₅-alkoxy)-2-butanol-Ester oder 4-Oxo-4-(hydroxy-C₃-C₅-alkoxy)-2-butanol-Ester einer polymeren Carbonsäure, insbesondere ein wie zuvor definierter Oxobutanol-Ester einer polymeren Carbonsäure, wobei der Oxobutanol-Ester der allgemeinen Formel (IIId) 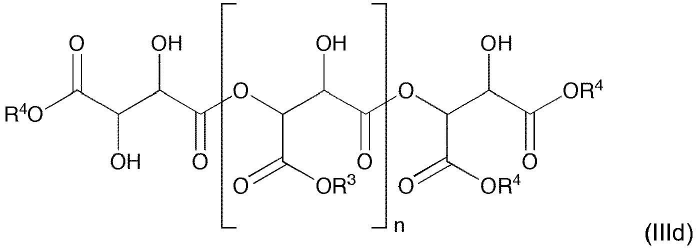 entspricht,
wobei in der allgemeinen Formel (Illd)
- die Variable n eine (ganze) Zahl ≥ 2, insbesondere im Bereich von zwei bis zwanzig, vorzugsweise im Bereich von zwei bis zehn, besonders bevorzugt im Bereich von drei bis sieben, darstellt;
- R³, unabhängig voneinander in Bezug auf die jeweilige Wiederholungseinheit, darstellt: Wasserstoff oder einen Rest - CH(CH₃) - CH₂ - C(O)OR¹, worin der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt;
- R⁴, unabhängig voneinander, Wasserstoff oder einen Rest - CH(CH₃) - CH₂ - C(O)OR¹ darstellt, worin der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt;
jedoch mit der Maßgabe, dass mindestens einer, insbesondere mindestens zwei, der Reste R³ und R⁴, einen Rest - CH(CH₃) - CH₂ - C(O)OR¹, wie zuvor definiert, darstellt, vorzugsweise mit der (weiteren) Maßgabe, dass mindestens ein Rest R⁴ einen Rest - CH(CH₃) - CH₂ - C(O)OR¹, wie zuvor definiert, darstellt.

Gegenstand der vorliegenden Erfindung gemäß einer besonderen Ausführungsform ist darüber hinaus ein Gemisch, umfassend mindestens zwei, insbesondere mindestens drei, voneinander verschiedene Oxobutanol-Ester einer polymeren Carbonsäure, insbesondere ein oder mehrere 4-Oxo-4-(C₁-C₅-alkoxy)-2-butanol-Ester oder 4-Oxo-4-(hydroxy-C₃-C₅-alkoxy)-2-butanol-Ester einer polymeren Carbonsäure, wie zuvor definiert.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, wie zuvor definiert, und/oder das nach dem erfindungsgemäßen Herstellungsverfahren erhältliche bzw. erfindungsgemäße Gemisch, wie zuvor definiert, weist gegenüber dem Stand der Technik eine Vielzahl von Vorteilen und Besonderheiten auf:

Wie die Anmelderin überraschend herausgefunden hat, eignet sich das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieser bzw. dieses einerseits physiologisch, insbesondere im Magen/Darm-Trakt, zu 3-Hydroxybuttersäure bzw. deren Salzen umgesetzt wird und andererseits gleichzeitig eine gute physiologische Kompatibilität bzw. Verträglichkeit aufweist, insbesondere im Hinblick auf Nichttoxizität und akzeptable organoleptische Eigenschaften.

Darüber hinaus ist das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, wie zuvor definiert, und/oder das nach dem erfindungsgemäßen Herstellungsverfahren erhältliche bzw. erfindungsgemäße Gemisch, wie zuvor definiert, erforderlichenfalls in enantiomerenreiner bzw. enantiomerenangereicherter Form bereitgestellt werden.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. Oxobutanol-Ester von polymeren Carbonsäuren, insbesondere 4-Oxo-4-(C₁-C₅-alkoxy)-2-butanol-Ester oder 4-Oxo-4-(hydroxy-C₃-C₅-alkoxy)-2-butanol-Ester von polymeren Carbonsäuren, wie zuvor definiert, und/oder das nach dem erfindungsgemäßen Herstellungsverfahren erhältliche bzw. erfindungsgemäße Gemisch, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) ein nach dem erfindungsgemäßen Herstellungsverfahren erhältliches bzw. erfindungsgemäßes Reaktionsprodukt, wie zuvor definiert, und/oder einen nach dem erfindungsgemäßen Herstellungsverfahren erhältlichen bzw. erfindungsgemäßen Oxobutanol-Ester von einer polymeren Carbonsäure, insbesondere einen 4-Oxo-4-(C₁-C₅-alkoxy)-2-butanol-Ester oder einen 4-Oxo-4-(hydroxy-C₃-C₅-alkoxy)-2-butanol-Ester von einer polymeren Carbonsäuren, wie zuvor definiert, und/oder ein nach dem erfindungsgemäßen Herstellungsverfahren erhältliches bzw. erfindungsgemäßes Gemisch, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **vierten** Aspekt der vorliegenden Erfindung - ist ein nach dem erfindungsgemäßen Herstellungsverfahren erhältliches bzw. erfindungsgemäßes Reaktionsprodukt, wie zuvor definiert, und/oder ein nach dem erfindungsgemäßen Herstellungsverfahren erhältlicher bzw. erfindungsgemäßer Oxobutanol-Ester von einer polymeren Carbonsäure, insbesondere 4-Oxo-4-(C₁-C₅-alkoxy)-2-butanol-Ester oder 4-Oxo-4-(hydroxy-C₃-C₅-alkoxy)-2-butanol-Ester von einer polymeren Carbonsäure, wie zuvor definiert, und/oder ein nach dem erfindungsgemäßen Herstellungsverfahren erhältliches bzw. erfindungsgemäßes Gemisch, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **fünften** Aspekt der vorliegenden Erfindung - ist die Verwendung eines nach dem erfindungsgemäßen Herstellungsverfahren erhältlichen bzw. erfindungsgemäßen Reaktionsprodukts, wie zuvor definiert, und/oder eines nach dem erfindungsgemäßen Herstellungsverfahren erhältlichen bzw. erfindungsgemäßen Oxobutanol-Esters von einer polymeren Carbonsäure, insbesondere eines 4-Oxo-4-(C₁-C₅-alkoxy)-2-butanol-Esters oder eines 4-Oxo-4-(hydroxy-C₃-C₅-alkoxy)-2-butanol-Esters von einer polymeren Carbonsäuren, wie zuvor definiert, und/oder eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem s**echsten** Aspekt der vorliegenden Erfindung - ist die Verwendung eines nach dem erfindungsgemäßen Herstellungsverfahren erhältlichen bzw. erfindungsgemäßen Reaktionsprodukts, wie zuvor definiert, und/oder eines nach dem erfindungsgemäßen Herstellungsverfahren erhältlichen bzw. erfindungsgemäßen Oxobutanol-Esters von einer polymeren Carbonsäure, insbesondere eines 4-Oxo-4-(C₁-C₅-alkoxy)-2-butanol-Esters oder eines 4-Oxo-4-(hydroxy-C₃-C₅-alkoxy)-2-butanol-Esters von einer polymeren Carbonsäure, wie zuvor definiert, und/oder eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

Gleichermaßen weiterer Gegenstand der vorliegenden Erfindung - gemäß einem **siebten** Aspekt der vorliegenden Erfindung - ist ein Nahrungsmittel- und/oder Lebensmittelerzeugnis, welches ein nach dem erfindungsgemäßen Herstellungsverfahren erhältliches bzw. erfindungsgemäßes Reaktionsprodukt, wie zuvor definiert, und/oder einen nach dem erfindungsgemäßen Herstellungsverfahren erhältlichen bzw. erfindungsgemäßen Oxobutanol-Ester von einer polymeren Carbonsäure, insbesondere einen 4-Oxo-4-(C₁-C₅-alkoxy)-2-butanol-Ester oder einen 4-Oxo-4-(hydroxy-C₃-C₅-alkoxy)-2-butanol-Ester von einer polymeren Carbonsäure, wie zuvor definiert, und/oder ein nach dem erfindungsgemäßen Herstellungsverfahren erhältliches bzw. erfindungsgemäßes Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Darüber hinaus kann auch die Verwendung eines nach dem erfindungsgemäßen Herstellungsverfahren erhältlichen Reaktionsprodukts, wie zuvor definiert, und/oder eines nach dem erfindungsgemäßen Herstellungsverfahren erhältlichen bzw. erfindungsgemäßen Oxobutanol-Esters von einer polymeren Carbonsäure, insbesondere eines 4-Oxo-4-(C₁-C₅-alkoxy)-2-butanol-Esters oder eines 4-Oxo-4-(hydroxy-C₃-C₅-alkoxy)-2-butanol-Esters von einer polymeren Carbonsäure, wie zuvor definiert, und/oder eines nach dem erfindungsgemäßen Herstellungsverfahren erhältlichen Gemischs, wie zuvor definiert, in einem Nahrungsmittel- und/oder Lebensmittelerzeugnis, vorgesehen sein.

In diesem Zusammenhang kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise der Ausgestaltung der vorliegenden Erfindung erläutern sollen.

### AUSFÜHRUNGSBEISPIELE:

**Verwendete Abkürzungen**

| | | |
|---|---|---|
| • | 3-BHB-EE=BHB-EE: | 3-Hydroxybuttersäureethylester (= Ethyl-3-hydroxybutanoat oder 4-Ethoxy-4-oxobutan-2-ol) (Edukt) |
| • | EE-BHB-PWS | Ethyl-3-hydroxybutanoat-Ester von Polyweinsäure |
| • | EE-BHB-PÄS | Ethyl-3-hydroxybutanoat-Ester von Polyäpfelsäure |
| • | EE-BHB-PCS | Ethyl-3-hydroxybutanoat-Ester von Polycitronensäure |

### I. Herstellungsbeispiele einer besonderen Ausführungsform des erfindungsgemäßen Verfahrens ohne die Verwendung eines Katalysators

### I. 1. Herstellung von 4-Ethoxy-4-oxobutan-2-ol-Estern (= Ethyl-3-hydroxybutanoat-Estern) von Metaweinsäure

In einem 100-ml-Mehrhalskolben mit Dephlegmator (Partialkondensator) und Destillationsbrücke werden 11 g (R)/(S)-3-Hydroxybuttersäureethylester (3-BHB-EE = Ethyl-3-hydroxybutanoat oder 4-Ethoxy-4-oxobutan-2-ol) (racemisch) und 5 g Metaweinsäure (Polyweinsäure) vorgelegt. Das Reaktionsgemisch wird unter Rühren bei 120 °C für 6 h zur Reaktion gebracht und das entstehende Reaktionswasser wird kontinuierlich destillativ entfernt. Eine Probe der Reaktionsmischung für weitere Analysen wird nach 3 h und nach 6 h genommen. Anschließend wird der überschüssige 3-Hydroxybuttersäureethylester im Stickstoffstrom bei 70 °C verdampft, dann wird eine weitere Probe genommen. Der erhaltene Rückstand wird für 2 bis 4 h im Hochvakuum dampfbehandelt.

Es wird ein Gemisch aus Ethyl-3-hydroxybutanoat-Mono-, Di- und höheren Estern von Metaweinsäure erhalten (d. h. also Gemisch von Metaweinsäure-mono(4-ethoxy-4-oxo-butan-2-ol)ester, Metaweinsäure-di(4-ethoxy-4-oxo-butan-2-ol)ester und Metaweinsäure-poly(4-ethoxy-4-oxo-butan-2-ol)ester).

Die Charakterisierung erfolgt mittels Gelpermeationschromatographie (GPC) und Säurezahlermittlung.

Die Säurezahl (SZ) gibt die Masse an Kaliumhydroxid (KOH) in Milligramm (mg) an, die erforderlich ist, um ein Gramm der zu analysierenden Säure (hier der Metaweinsäure) zu neutralisieren. Die Säurezahl ist ein Maß der Carbonsäuregruppen in einer chemischen Verbindung und wird zur Quantifizierung des Säuregehalts einer Substanz verwendet.

Die Säurezahl der reinen Metaweinsäure (Edukt) liegt bei 469 mg KOH/g und die Säurezahl des erhaltenen Reaktionsprodukts liegt bei 190 mg KOH/g. Somit ist die Anzahl der freien Carbonsäuregruppen signifikant gesunken, sodass eine erfolgreiche Veresterung mit 3-Hydroxybuttersäureethylester stattgefunden hat.

Die im Laufe des Syntheseverfahrens genommenen Proben (nach 3 h Reaktionszeit sowie nach 6 h Reaktionszeit und nach der Stickstoffverdampfung) sowie die reine Metaweinsäure (Edukt) werden mittels Gelpermeationschromatographie (GPC) analysiert; in Tabelle 1 sind die GC-Flächenanalysen zusammengefasst:

**Tabelle 1: GC-Flächenanalyse [%] der Umsetzung von Metaweinsäure mit Ethyl-3-hydroxybutanoat**

| **Metaweinsäure** | | **EE-BHB-PWS** | | **EE-BHB-PWS** | | **EE-BHB-PWS** | |
|---|---|---|---|---|---|---|---|
| **M [g/mol]** | **GPC-Fläche [%]** | **M [g/mol]** | **GPC-Fläche [%]** | **M [g/mol]** | **GPC-Fläche [%]** | **M [g/mol]** | **GPC-Fläche [%]** |
| 150 * | 3,69 | 150 * | 2,36 | 150 * | 2,05 | 150 * | 0,59 |
| - | - | 190 ** | 27,43 | 190 ** | 18,81 | 190 ** | 3,17 |
| - | - | - | - | 230 | 3,05 | 230 | 3,21 |
| 360 | 23,6 | 340 | 11,08 | 320 | 12,33 | 320 | 13,34 |
| - | - | 440 | 5,64 | 440 | 7,49 | 440 | 10,13 |
| 570 | 17,33 | 550 | 5,96 | 550 | 6,13 | 550 | 7,42 |
| 740 | 14,17 | 690 | 7,04 | 690 | 7,26 | 690 | 8,59 |
| 890 | 14,76 | 850 | 6,6 | 850 | 6,67 | 850 | 8,14 |
| 960 | 24,97 | 1000 | 33,58 | 1000 | 36,21 | 990 | 45,41 |
| **Mw/Mn** | 710/530 | | 890/390 | | 980/430 | | 1200/610 |
| **PDI** | 1,34 | | 2,28 | | 2,28 | | 1,97 |

| | | | | | | | |
|---|---|---|---|---|---|---|---|
| * Weinsäure ** 3-BHB-EE | | | | | | | |

Im Folgenden ist der Reaktionsverlauf schematisch dargestellt:

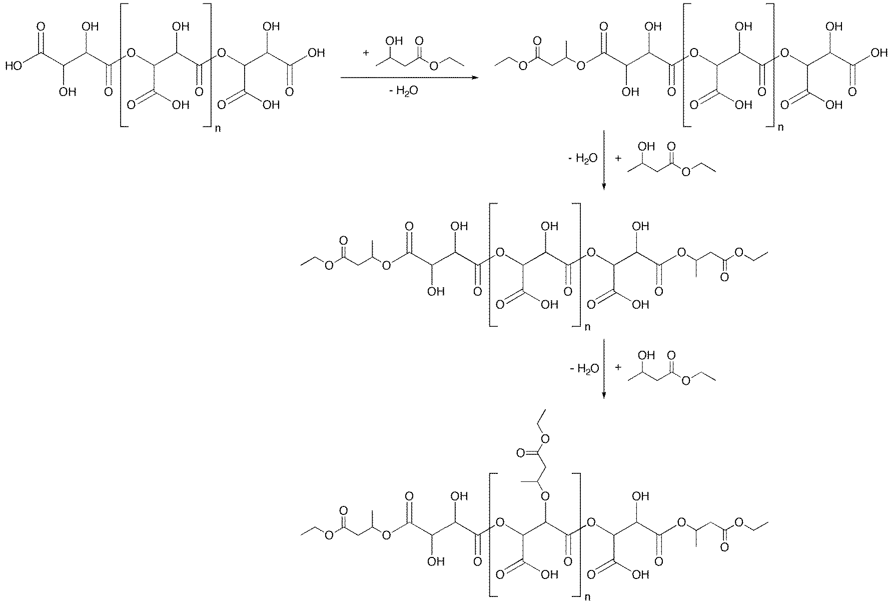

### I. 2. Herstellung von 4-Ethoxy-4-oxobutan-2-ol-Estern (= Ethyl-3-hydroxybutanoat-Estern) von Polyäpfelsäure

In einem 100-ml-Mehrhalskolben mit Dephlegmator (Partialkondensator) und Destillationsbrücke werden 11 g (R)/(S)-3-Hydroxybuttersäureethylester (3-BHB-EE = Ethyl-3-hydroxybutanoat oder 4-Ethoxy-4-oxobutan-2-ol) (racemisch) und 5 g Polyäpfelsäure vorgelegt. Das Reaktionsgemisch wird unter Rühren bei 120 °C für 6 h zur Reaktion gebracht und das entstehende Reaktionswasser wird kontinuierlich destillativ entfernt. Anschließend wird der überschüssige 3-Hydroxybuttersäureethylester im Stickstoffstrom bei 70 °C verdampft und anschließend wird eine weitere Probe genommen. Der erhaltene Rückstand wird für 2 bis 4 h im Hochvakuum dampfbehandelt.

Es wird ein Gemisch aus Ethyl-3-hydroxybutanoat-Mono-, Di- und höheren Estern von Polyäpfelsäure erhalten.

Die Charakterisierung erfolgt mittels Gelpermeationschromatographie (GPC) und Säurezahlermittlung.

### I. 3. Herstellung von 4-Ethoxy-4-oxobutan-2-ol-Estern (= Ethyl-3-hydroxybutanoat-Estern) von Polycitronensäure

In einem 100-ml-Mehrhalskolben mit Dephlegmator (Partialkondensator) und Destillationsbrücke werden 11 g (R)/(S)-3-Hydroxybuttersäureethylester (3-BHB-EE = Ethyl-3-hydroxybutanoat oder 4-Ethoxy-4-oxobutan-2-ol) (racemisch) und 5 g Polycitronensäure vorgelegt. Das Reaktionsgemisch wird unter Rühren bei 120 °C für 6 h zur Reaktion gebracht und das entstehende Reaktionswasser wird kontinuierlich destillativ entfernt. Anschließend wird der überschüssige 3-Hydroxybuttersäureethylester im Stickstoffstrom bei 70 °C verdampft und anschließend wird eine weitere Probe genommen. Der erhaltene Rückstand wird für 2 bis 4 h im Hochvakuum dampfbehandelt.

Es wird ein Gemisch aus Ethyl-3-hydroxybutanoat-Mono-, Di- und höheren Estern von Polycitronensäure erhalten.

Die Charakterisierung erfolgt mittels Gelpermeationschromatographie (GPC) und Säurezahlermittlung.

### I. 4. Herstellung weiterer 4-Ethoxy-4-oxobutan-2-ol-Ester (= Ethyl-3-hydroxybutanoat-Ester) von polymeren Carbonsäuren

Darüber hinaus werden die vorgenannten Synthesen Nr. I. 1, I. 2 und I. 3 jeweils wiederholt, jedoch jeweils mit anderen 3-Hydroxybutanoaten (nämlich jeweils 3-Hydroxybuttersäure-(hydroxybutyl)-ester bzw. 3-Hydroxybuttersäure-(hydroxypentyl)-ester anstelle von 3-Hydroxybuttersäureethylester). 3-Hydroxybuttersäure-(hydroxybutyl)-ester wird durch Veresterung von 3-Hydroxybuttersäure mit Butandiol (z. B. mit 1,3-Butandiol) erhalten, während 3-Hydroxybuttersäure-(hydroxypentyl)-ester durch Veresterung von 3-Hydroxybuttersäure mit Pentandiol (z. B. mit 1,3-Pentandiol) erhalten wird. Es werden vergleichbare Ergebnisse erhalten. Aufreinigung und Trennung erfolgen in gleicher Weise.

Auch werden die zuvor genannten Herstellungsbeispiele wiederholt, jedoch mit den Methyl- und Ethylestern der polymeren Carbonsäuren. Es werden vergleichbare Ergebnisse erhalten. Aufreinigung und Trennung erfolgen in gleicher Weise.

### II. Herstellungsbeispiele einer alternativen besonderen Ausführungsform des erfindungsgemäßen Verfahrens unter Verwendung eines Metallkatalysators

Alle zuvor unter Abschnitt I. beschriebenen chemischen Synthesebeispiele werden erneut durchgeführt, jedoch unter Zugabe von Titantetrabutylat als Katalysator (Titan(IV)-Katalysator). Der Titan(IV)-Katalysator wird zusammen mit den weiteren Reaktanden im Kolben vorgelegt. Im Anschluss entspricht der Reaktionsverlauf den zuvor beschriebenen Beispielen. Es werden vergleichbare Ergebnisse erzielt. Der Katalysator wird nach Reaktionsende abgetrennt und rezykliert.

### III. Herstellungsbeispiele einer weiteren alternativen besonderen Ausführungsform des erfindungsgemäßen Verfahrens unter Verwendung eines Enzymkatalysators

Alle zuvor unter Abschnitt I. beschriebenen chemischen Synthesebeispiele werden erneut durchgeführt, jedoch unter Zugabe eines immobilisierten Enzyms (CALB-Lipase auf Polymerträger, abgeleitet von *Candida antarctica,* z. B. Novozym^{®}435 von der Fa. Sigma-Aldrich bzw. Merck oder Lipozym^{®}435 von der Fa. Strem Chemicals, Inc.) als Katalysators und bei 70 °C unter Vakuum. Es werden vergleichbare Ergebnisse erzielt. Der Katalysator (d. h. das Enzym) wird nach Reaktionsende abgetrennt und rezykliert.

### IV. Physiologische Anwendunasversuche: in-vitro-Verdauversuche

### Verdauversuche (Spalt- bzw. Spaltungsversuche) von erfindungsgemäßen 3-Hydroxybutanoat-Estern von polymeren Carbonsäuren

Mittels Spaltungsversuchen wird gezeigt, dass erfindungsgemäß hergestellte 3-Hydroxybutanoat-Ester von polymeren Carbonsäuren bzw. deren Gemische (vgl. zuvor beschriebene Versuche gemäß I., II. und III.), einschließlich der Reaktionsnebenprodukte, im menschlichen gastrointestinalen Trakt gespalten werden können.

Als Ausgangsgemisch werden jeweils aufgereinigte, nach dem erfindungsgemäßen Verfahren erhaltene Reaktionsprodukte eingesetzt (d. h. 3-Hydroxybutanoat-Ester von Polyweinsäure, 3-Hydroxybutanoat-Ester von Polyäpfelsäure und 3-Hydroxybutanoat-Ester von Polycitronensäure).

Für die Spaltungsversuche unter köpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Bei den Spaltungsversuchen ist zu erkennen, dass die Abspaltung von 3-Hydroxybutanoat in einer Kaskade verläuft (d. h. das das 3-Hydroxybutanoat schrittweise freigesetzt wird und nachfolgend weiterführend zu der freien 3-Hydroxybuttersäure und Ethanol gespalten werden kann).

### Weitere Verdauversuche (Spaltunasversuche) von erfindungsgemäßen Carbonsäuren von 3-Hydroxybutanoat

### Spaltunqsversuche mit Pankreatin

2 g eines wie zuvor beschrieben hergestellten 3-Hydroxybutanoat-Esters einer polymeren Carbonsäure bzw. eine entsprechende Mischung werden in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung der 3-Hydroxybutanoat-Ester von polymeren Carbonsäuren zu dem freien 3-Hydroxybuttanoat). Der Umsatz/Zeit-Verlauf der wässrigen Spaltung der erfindungsgemäßen 3-Hydroxybutanoat-Ester der polymeren Carbonsäuren mittels Pankreatin, einschließlich Zunahme der Säurezahl über die Zeit, belegt die gewünschte Zersetzung des Eduktgemischs zu der freien polymeren Carbonsäure und dem 3-Hydroxybutanoat. Dies wird durch entsprechende Analytik bestätigt. Der Versuch belegt, dass das erfindungsgemäße Ausgangsgemisch ein geeigneter physiologischer Präkursor für 3-Hydroxybuttersäure bzw. deren Ester (3-Hydroxybutanoate) für die entsprechenden Ketokörpertherapien darstellt.

Der Versuch wird jeweils anhand der einzelnen Ester in Reinform durchgeführt und verifiziert. Es werden jeweils vergleichbare Ergebnisse erhalten, d. h. die 3-Hydroxybutanoat-Ester von polymeren Carbonsäuren werden durch Pankreatin jeweils zu der freien polymeren Carbonsäure und 3-Hydroxybutanoat gespalten.

Die zuvor geschilderten Spaltungsversuche belegen, dass die 3-Hydroxybutanoat-Ester von polymeren Carbonsäuren effiziente Präkursoren bzw. Metabolite der freien Hydroxybuttersäure bzw. deren Estern (hier: Ethylester, Hydroxybutylester und Hydroxypentylester) darstellen, insbesondere im Hinblick auf ihre beabsichtigte Wirkung, welche in physiologisch verträglicher bzw. physiologisch kompatibler Form vorliegen. Gleichermaßen werden ebenfalls metabolisch verwertbare bzw. umsetzbare, auf im natürlichen Stoffwechsel (z. B. Citratzyklus) vorkommende Carbonsäuren zugrundeliegende polymeren Carbonsäuren, gebildet (z. B. Polycitronensäure bzw. Polycitrate, Polyäpfelsäure bzw. Polymalate, Polyweinsäure bzw. Metaweinsäure bzw. Polytartrate etc.).

### VI. Weitere Testungen (Organoleptik und Toxizität)

Weitere Versuche und Testreihen werden in Bezug auf Organoleptik und Toxizität der erfindungsgemäßen 3-Hydroxybutanoat-Ester von polymeren Carbonsäuren durchgeführt. Diese zeigen, dass die erfindungsgemäßen 3-Hydroxybutanoat-Ester von polymeren Carbonsäuren organoleptisch akzeptabel und kompatibel sind, insbesondere im Vergleich zu reiner 3-Hydroxybuttersäure sowie deren Salzen und Estern signifikant verbesserte organoleptische Eigenschaften aufweisen, sowie zudem keine für die Anwendung entgegenstehende Toxizität aufweisen.

## Patentansprüche

1. Verfahren zur Herstellung von Oxobutanol-Estern von polymeren Carbonsäuren,
wobei mindestens ein Oxobutanol der allgemeinen Formel (I)
CH₃ - CH(OH) - CH₂ - C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer wenigstens drei Carboxylgruppen enthaltenden polymeren, insbesondere polykondensierten oder polymerisierten, Carbonsäure (II), ausgewählt aus der Gruppe von Polyweinsäure, Polyäpfelsäure und Polycitronensäure sowie deren Salzen und partiellen Salzen und deren Estern und partiellen Estern und deren Kombinationen und Mischungen, in einer Veresterungsreaktion und/oder unter Veresterungsbedingungen umgesetzt wird,
so dass als Reaktionsprodukt (III) ein oder mehrere Oxobutanol-Ester der polymeren Carbonsäure (II) erhalten wird/werden.

2. Verfahren nach Anspruch 1,
wobei die Umsetzung in Abwesenheit von Lösemitteln und/oder ohne jedwedes Lösemittel durchgeführt wird; und
wobei die Umsetzung in Abwesenheit eines Katalysators und/oder ohne jedweden Katalysator durchgeführt wird.

3. Verfahren nach Anspruch 1 oder Anspruch 2,
wobei die Umsetzung in Abwesenheit eines Katalysator und/oder ohne jedweden Katalysator durchgeführt wird;
insbesondere wobei die Umsetzung in Abwesenheit eines Katalysator und/oder ohne jedweden Katalysator bei Temperaturen im Bereich von 20 °C bis 160 °C, insbesondere im Bereich von 50 °C bis 150°C, vorzugsweise im Bereich von 70 °C bis 140°C, besonders bevorzugt im Bereich von 80 °C bis 135 °C, ganz besonders bevorzugt im Bereich von 100 °C bis 130 °C, durchgeführt wird; und/oder
insbesondere wobei die Umsetzung in Abwesenheit eines Katalysator und/oder ohne jedweden Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird; und/oder
insbesondere wobei die Umsetzung in Abwesenheit eines Katalysator und/oder ohne jedweden Katalysator in Gegenwart eines Inertgases, insbesondere in Gegenwart von Helium, Argon oder Stickstoff, bevorzugt in Gegenwart von Stickstoff, durchgeführt wird.

4. Verfahren nach Anspruch 1 oder Anspruch 2,
wobei die Umsetzung in Gegenwart eines Enzyms als Katalysator durchgeführt wird;
insbesondere wobei das Enzym ausgewählt wird aus Synthetasen (Ligasen), Katalasen, Esterasen, Lipasen und deren Kombinationen;
insbesondere wobei das Enzym sich ableitet von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus;* und/oder
insbesondere wobei das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird; und/oder
insbesondere wobei das Enzym nach der Umsetzung rezykliert wird; und/oder
insbesondere wobei die Umsetzung in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird; und/oder
insbesondere wobei das Enzym in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt wird; und/oder
insbesondere wobei die Umsetzung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 0,5 bar, durchgeführt wird; und/oder
insbesondere wobei die Umsetzung in Gegenwart eines Enzyms in Gegenwart eines Inertgases, insbesondere in Gegenwart von Helium, Argon oder Stickstoff, bevorzugt in Gegenwart von Stickstoff, durchgeführt wird.

5. Verfahren nach Anspruch 1 oder Anspruch 2,
wobei die Umsetzung in Gegenwart eines metallhaltigen und/oder metallbasierten, sauren oder basischen Katalysators durchgeführt wird;
insbesondere wobei der Katalysator ausgewählt ist aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen; und/oder
insbesondere wobei als Katalysator eine Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl, eingesetzt wird; und/oder
insbesondere wobei der Katalysator nach der Umsetzung rezykliert wird; und/oder insbesondere wobei die Umsetzung in Gegenwart eines metallhaltigen und/oder metallbasierten, sauren oder basischen Katalysators bei Temperaturen im Bereich von 20 °C bis 160 °C, insbesondere im Bereich von 50 °C bis 150 °C, vorzugsweise im Bereich von 70 °C bis 140 °C, besonders bevorzugt im Bereich von 80 °C bis 135 °C, ganz besonders bevorzugt im Bereich von 100 °C bis 130 °C, durchgeführt wird; und/oder
insbesondere wobei der Katalysator in Mengen, bezogen auf die Gesamtmenge der Ausgangsverbindungen (I) und (II), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt wird; und/oder
insbesondere wobei die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird; und/oder
insbesondere wobei die Umsetzung in Gegenwart eines metallhaltigen und/oder metallbasierten, sauren oder basischen Katalysators in Gegenwart eines Inertgases, insbesondere in Gegenwart von Helium, Argon oder Stickstoff, bevorzugt in Gegenwart von Stickstoff, durchgeführt wird.

6. Verfahren nach einem der vorangehenden Ansprüche,
wobei das Oxobutanol der allgemeinen Formel (I), bezogen auf die Carboxylgruppen der polymeren Carbonsäure (II) in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei das Oxobutanol der allgemeinen Formel (I) und die polymere Carbonsäure (II) in einem Molverhältnis von Oxobutanol der allgemeinen Formel (I) / Carboxylgruppen der polymeren Carbonsäure (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

7. Verfahren nach einem der vorangehenden Ansprüche,
wobei im Reaktionsprodukt nach erfolgter Umsetzung noch vorhandene Hydroxylgruppen und/oder Carboxylgruppen zumindest teilweise, vorzugsweise vollständig, funktionalisiert, insbesondere verestert werden; und/oder
wobei sich der Umsetzung eine teilweise, insbesondere vollständige Funktionalisierung, insbesondere Veresterung, noch vorhandener Hydroxylgruppen und/oder Carboxylgruppen anschließt.

8. Reaktionsprodukt, erhältlich gemäß dem Verfahren nach einem der vorangehenden Ansprüche.

9. Oxobutanol-Ester einer polymeren Carbonsäure,
wobei die polymere Carbonsäure ausgewählt ist aus der Gruppe von Polyweinsäure, Polyäpfelsäure und Polycitronensäure sowie deren Salzen und partiellen Salzen und Estern und partiellen Estern und deren Kombinationen und Mischungen; und
wobei mindestens eine Carboxylgruppe, insbesondere mindestens eine endständige und/oder primäre Carboxylgruppe, der polymeren Carbonsäure verestert ist mit einem Oxobutanol der allgemeinen Formel (I)
CH₃ - CH(OH) - CH₂ - C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt.

10. Oxobutanol-Ester einer polymeren Carbonsäure nach Anspruch 9,
wobei die polymere Carbonsäure der allgemeinen Formel (IId) 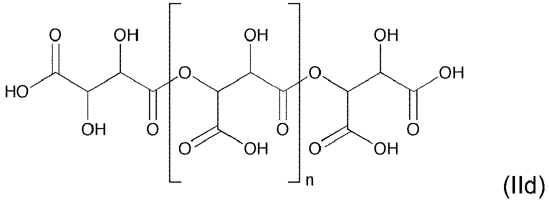 entspricht,
wobei in der allgemeinen Formel (IId) die Variable n eine (ganze) Zahl ≥ 2, insbesondere im Bereich von zwei bis zwanzig, vorzugsweise im Bereich von zwei bis zehn, besonders bevorzugt im Bereich von drei bis sieben, darstellt.

11. Oxobutanol-Ester einer polymeren Carbonsäure nach Anspruch 9 oder Anspruch 10, wobei der Oxobutanol-Ester der allgemeinen Formel (IIId) 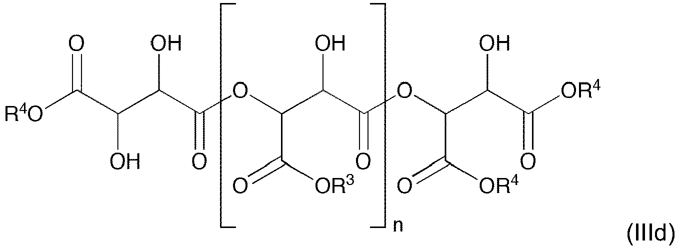 entspricht,
wobei in der allgemeinen Formel (IIId)
• die Variable n eine (ganze) Zahl ≥ 2, insbesondere im Bereich von zwei bis zwanzig, vorzugsweise im Bereich von zwei bis zehn, besonders bevorzugt im Bereich von drei bis sieben, darstellt;
• R³, unabhängig voneinander in Bezug auf die jeweilige Wiederholungseinheit, darstellt: Wasserstoff oder einen Rest - CH(CH₃) - CH₂ - C(O)OR¹, worin der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt;
• R⁴, unabhängig voneinander, Wasserstoff oder einen Rest - CH(CH₃) - CH₂ - C(O)OR¹ darstellt, worin der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt;
jedoch mit der Maßgabe, dass mindestens einer, insbesondere mindestens zwei, der Reste R³ und R⁴, einen Rest - CH(CH₃) - CH₂ - C(O)OR¹, wie zuvor definiert, darstellt, vorzugsweise mit der (weiteren) Maßgabe, dass mindestens ein Rest R⁴ einen Rest - CH(CH₃) - CH₂ - C(O)OR¹, wie zuvor definiert, darstellt.

12. Gemisch, umfassend mindestens zwei, insbesondere mindestens drei, voneinander verschiedene Oxobutanol-Ester einer polymeren Carbonsäure, wie in vorangehenden Ansprüchen 8 bis 11 definiert.

13. Pharmazeutische Zusammensetzung, insbesondere Arzneimittel oder Medikament, umfassend ein Reaktionsprodukt gemäß Anspruch 8 und/oder einen Oxobutanol-Ester von einer polymeren Carbonsäure gemäß einem der Ansprüche 9 bis 11 und/oder ein Gemisch gemäß Anspruch 12.

14. Pharmazeutische Zusammensetzung nach Anspruch 13 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

15. Reaktionsprodukt gemäß Anspruch 8 und/oder Oxobutanol-Ester von einer polymeren Carbonsäure gemäß einem der Ansprüche 9 bis 11 und/oder Gemisch gemäß Anspruch 12 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

16. Verwendung eines Reaktionsprodukts gemäß Anspruch 8 und/oder eines Oxobutanol-Esters von einer polymeren Carbonsäure gemäß einem der Ansprüche 9 bis 11 und/oder eines Gemischs gemäß Anspruch 12 zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

17. Verwendung eines Reaktionsprodukts gemäß Anspruch 8 und/oder eines Oxobutanol-Esters von einer polymeren Carbonsäure gemäß einem der Ansprüche 9 bis 11 und/oder eines Gemischs gemäß Anspruch 12 zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

18. Nahrungsmittel- und/oder Lebensmittelerzeugnis, umfassend ein Reaktionsprodukt gemäß Anspruch 8 und/oder einen Oxobutanol-Ester von einer polymeren Carbonsäure gemäß einem der Ansprüche 9 bis 11 und/oder ein Gemisch gemäß Anspruch 12.

## Claims

1. A method for producing oxobutanol esters of polymeric carboxylic acids,
wherein at least one oxobutanol of general formula (I)
CH₃ - CH(OH) - CH₂ - C(O)OR¹ (I)
wherein, in the general formula (I), the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl,
is reacted in an esterification reaction and/or under esterification conditions with at least one polymeric, especially polycondensed or polymerized, carboxylic acid (II) comprising at least three carboxyl groups and selected from the group consisting of polytartaric acid, polymalic acid and polycitric acid as well as their salts and partial salts and esters and partial esters and combinations and mixtures thereof,
so that, as a reaction product (III), one or more oxobutanol esters of the polymeric carboxylic acid (II) is/are obtained.

2. The method according to claim 1,
wherein the reaction is carried out in the absence of solvents and/or without any solvent; and
wherein the reaction is carried out in the absence of a catalyst and/or without any catalyst.

3. The method according to claim 1 or claim 2,
wherein the reaction is carried out in the absence of a catalyst and/or without any catalyst;
especially wherein the reaction is carried out in the absence of a catalyst and/or without any catalyst at temperatures in the range of from 20 °C to 160 °C, especially in the range of from 50 °C to 150 °C, preferentially in the range of from 70 °C to 140 °C, more preferably in the range of from 80 °C to 135 °C, even more preferably in the range of from 100 °C to 130 °C; and/or
especially wherein the reaction is carried out in the absence of a catalyst and/or without any catalyst at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar; and/or
especially wherein the reaction is carried out in the absence of a catalyst and/or without any catalyst in the presence of an inert gas, especially in the presence of helium, argon or nitrogen, preferably in the presence of nitrogen.

4. The method according to claim 1 or claim 2,
wherein the reaction is carried out in the presence of an enzyme as a catalyst;
especially wherein the enzyme is selected from synthetases (ligases), catalases, esterases, lipases and combinations thereof; and/or
especially wherein the enzyme is derived from *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* and *Pseudomonas sp.* and combinations thereof, preferentially of *Candida antarctica, Mucor miehei (Rhizomucor miehei)* and *Thermomyces lanuginosus;* and/or
especially wherein the enzyme is used in immobilized form, especially immobilized on a carrier, preferentially on a polymeric carrier, preferably on a polymeric organic carrier, more preferably with hydrophobic properties, even more preferably on a poly(meth)acrylic resin-based carrier; and/or
especially wherein the enzyme is recycled after the reaction; and/or
especially wherein the reaction is carried out in the presence of an enzyme as a catalyst at temperatures in the range of from 10 °C to 80 °C, especially in the range of from 20 °C to 80 °C, preferentially in the range of from 25 °C to 75 °C, more preferably in the range of from 45 °C to 75 °C, even more preferably in the range of from 50 °C to 70 °C; and/or
especially wherein the enzyme is used in amounts, based on the total amount of starting compounds (I) and (II), in the range of from 0.001 % by weight to 20 % by weight, especially in the range of from 0.01 % by weight to 15 % by weight, preferentially in the range of from 0.1 % by weight to 15 % by weight, preferably in the range of from 0.5 % by weight to 10 % by weight; and/or
especially wherein the reaction is carried out in the presence of an enzyme as a catalyst at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 0.5 bar; and/or
especially wherein the reaction is carried out in the presence of an enzyme in the presence of an inert gas, especially in the presence of helium, argon or nitrogen, preferably in the presence of nitrogen.

5. The method according to claim 1 or claim 2,
wherein the reaction is carried out in the presence of a metal-containing and/or metal-based, acidic or basic catalyst;
especially wherein the catalyst is selected from (i) basic catalysts, especially alkali or alkaline earth hydroxides and alkali or alkaline earth alcoholates, such as NaOH, KOH, LiOH, Ca(OH)₂, NaOMe, KOMe and Na(OBu-tert.), (ii) acidic catalysts, especially mineral acids, and organic acids, such as sulfuric acid, hydrochloric acid, phosphoric acid, nitric acid, sulfonic acids, methane sulfonic acid, para-toluene sulfonic acid and carboxylic acids, (iii) Lewis acids, especially Lewis acids based on titanium, tin, zinc and aluminum compounds, such as titanium tetrabutylate, tin acids, zinc acetate, aluminum trichloride and aluminum tri-isopropyl, and (iv) heterogeneous catalysts, especially based on mineral silicates, germanates, carbonates and aluminum oxides, such as zeolites, montmorillonites, mordenites, hydrotalcites and aluminas, and combinations thereof; and/or
especially wherein a Lewis acid based on titanium, tin, zinc and aluminum compounds, such as titanium tetrabutylate, tin acids, zinc acetate, aluminum trichloride and aluminum tri-isopropyl, is used as a catalyst; and/or
especially wherein the catalyst is recycled after the reaction; and/or
especially wherein the reaction is carried out in the presence of a metal-containing and/or metal-based, acidic or basic catalyst at temperatures in the range of from 20 °C to 160 °C, especially in the range of from 50 °C to 150 °C, preferentially in the range of from 70 °C to 140 °C, more preferably in the range of from 80 °C to 135 °C, even more preferably in the range of from 100 °C to 130 °C; and/or
especially wherein the catalyst is used in amounts, based on the total amount of starting compounds (I) and (II), in the range of from 0.01 % by weight to 30 % by weight, especially in the range of from 0.05 % by weight to 15 % by weight, preferentially in the range of from 0.1 % by weight to 15 % by weight, preferably in the range of from 0.2 % by weight to 10 % by weight; and/or
especially wherein the reaction is carried out in the presence of a metal-containing and/or metal-based, acidic or basic catalyst at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar; and/or
especially wherein the reaction is carried out in the presence of a metal-containing and/or metal-based, acidic or basic catalyst in the presence of an inert gas, especially in the presence of helium, argon or nitrogen, preferably in the presence of nitrogen.

6. The method according to any of the preceding claims,
wherein the oxobutanol of the general formula (I), based on the carboxyl groups of the polymeric carboxylic acid (II), is used in molar amounts in a range of from equimolar amount up to a molar excess of 200 mol-%, especially in a range of from equimolar amount up to a molar excess of 150 mol-%, preferentially in a range of from equimolar amount up to a molar excess of 100 mol-%; and/or
wherein the oxobutanol of the general formula (I) and the polymeric carboxylic acid (II) are used in a molar ratio of oxobutanol of the general formula (I) / carboxyl groups of the polymeric carboxylic acid (II) in a range of from 1 : 1 to 10 : 1, especially in a range of from 2 : 1 to 8 : 1, preferentially in a range of from 3 : 1 to 6 : 1.

7. The method according to any of the preceding claims,
wherein hydroxyl groups and/or carboxyl groups still present in the reaction product after the reaction has been performed are at least partially, preferentially completely, functionalized, especially esterified; and/or
wherein the reaction is followed by a partial, especially complete functionalization, especially esterification, of hydroxyl groups and/or carboxyl groups still present.

8. A reaction product obtainable by the method according to any of the preceding claims.

9. An oxobutanol ester of a polymeric carboxylic acid,
wherein the polymeric carboxylic acid is selected from the group consisting of polytartaric acid, polymalic acid and polycitric acid as well as their salts and partial salts and esters and partial esters and combinations and mixtures thereof; and
wherein at least one carboxyl group, especially at least one terminal and/or primary carboxyl group, of the polymeric carboxylic acid is esterified with an oxobutanol of the general formula (I)
CH₃ - CH(OH) - CH₂ - C(O)OR¹ (I)
wherein, in the general formula (I), the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl.

10. The oxobutanol ester of a polymeric carboxylic acid according to claim 9,
wherein the polymeric carboxylic acid corresponds to general formula (IId) 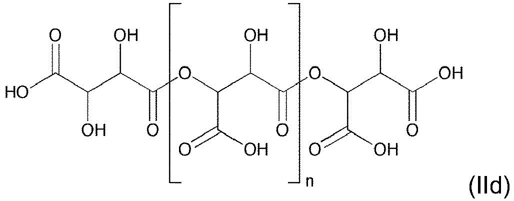
wherein, in the general formula (IId), the variable n represents an (integer) number ≥ 2, especially in the range of from two to twenty, preferentially in the range of from two to ten, more preferably in the range of from three to seven.

11. An oxobutanol ester of a polymeric carboxylic acid according to claim 9 or claim 10,
wherein the oxobutanol ester corresponds to general formula (IIId) 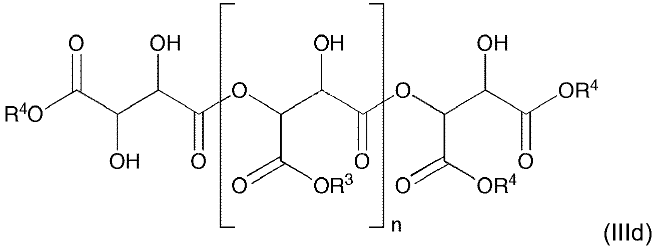
wherein, in the general formula (IIId),
• the variable n represents an (integer) number ≥ 2, especially in the range of from two to twenty, preferentially in the range of from two to ten, more preferably in the range of from three to seven;
• R³, independently of one other with respect to the respective repeating unit, represents: hydrogen or a radical - CH(CH₃) - CH₂ - C(O)OR¹, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl;
• R⁴, independently of one another, represents hydrogen or a radical - CH(CH₃) - CH₂ - C(O)OR¹, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl;
however, with the proviso that at least one, especially at least two, of the radicals R³ and R⁴ represents a radical - CH(CH₃) - CH₂ - C(O)OR¹, as defined above, preferentially with the (further) proviso that at least one radical R⁴ represents a radical - CH(CH₃) - CH₂ - C(O)OR¹, as defined above.

12. A mixture comprising at least two, especially at least three, different oxobutanol esters of a polymeric carboxylic acid, as defined above.

13. A pharmaceutical composition, especially a drug or medicament, comprising a reaction product according to claim 8 and/or an oxobutanol ester of a polymeric carboxylic acid according to any of claims 9 to 11 and/or a mixture according to claim 12.

14. The pharmaceutical composition according to claim 13 for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

15. A reaction product according to claim 8 and/or an oxobutanol ester of a polymeric carboxylic acid according to any of claims 9 to 11 and/or a mixture according to claim 12 for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

16. Use of a reaction product according to claim 8 and/or an oxobutanol ester of a polymeric carboxylic acid according to any of claims 9 to 11 and/or a mixture according to claim 12 for producing a medicament for the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

17. Use of a reaction product according to claim 8 and/or an oxobutanol ester of a polymeric carboxylic acid according to any of claims 9 to 11 and/or a mixture according to claim 12 for producing a medicament for the prophylactic and/or therapeutic treatment of or for the application for catabolic metabolic states, such as hunger, diets or low-carbohydrate nutrition.

18. A food and/or a food product comprising a reaction product according to claim 8 and/or an oxobutanol ester of a polymeric carboxylic acid according to any of claims 9 to 11 and/or a mixture according to claim 12.

## Revendications

1. Procédé de préparation d'esters d'oxobutanol d'acides carboxyliques polymères,
où l'on fait réagir, dans une réaction d'estérification et/ou dans des conditions d'estérification, au moins un oxobutanol de formule générale (I)
CH₃ - CH(OH) - CH₂ - C(O)OR¹ (I)
où, dans la formule générale (I), le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle,
avec au moins un acide carboxylique (II) polymère, en particulier polycondensé ou polymérisé, contenant au moins trois groupes carboxyle et étant choisi dans le groupe de l'acide polytartrique, de l'acide polymalique et de l'acide polycitrique ainsi que leurs sels et sels partiels et leurs esters et esters partiels et leurs combinaisons et mélanges,
de sorte que l'on obtient, comme produit de réaction (III), un ou plusieurs esters d'oxobutanol de l'acide carboxylique polymère (II).

2. Procédé selon la revendication 1,
où la réaction est effectuée en l'absence de solvants et/ou sans aucun solvant; et
où la réaction est effectuée en l'absence de catalyseur et/ou sans aucun catalyseur.

3. Procédé selon la revendication 1 ou la revendication 2,
où la réaction est effectuée en l'absence de catalyseur et/ou sans aucun catalyseur;
en particulier où la réaction est effectuée en l'absence d'un catalyseur et/ou sans aucun catalyseur à des températures dans la plage de 20 °C à 160 °C, en particulier dans la plage de 50 °C à 150 °C, de préférence dans la plage de 70 °C à 140 °C, de manière particulièrement préférée dans la plage de 80 °C à 135 °C, de manière tout particulièrement préférée dans la plage de 100 °C à 130 °C; et/ou
en particulier où la réaction est effectuée en l'absence d'un catalyseur et/ou sans aucun catalyseur à une pression dans la plage de 0,0001 bar à 10 bars, en particulier dans la plage de 0,001 bar à 5 bars, de préférence dans la plage de 0,01 bar à 2 bars, de manière particulièrement préférée dans la plage de 0,05 bar à 1 bar, tout particulièrement à environ 1 bar; et/ou
en particulier où la réaction est effectuée en l'absence d'un catalyseur et/ou sans aucun catalyseur en présence d'un gaz inerte, en particulier en présence d'hélium, d'argon ou d'azote, de préférence en présence d'azote.

4. Procédé selon la revendication 1 ou la revendication 2,
où la réaction est effectuée en présence d'une enzyme servant de catalyseur;
en particulier où l'enzyme est choisie parmi les synthétases (ligases), les catalases, les estérases, les lipases et leurs combinaisons;
en particulier où l'enzyme est dérivée de *Candida antarctica, Mucor miehei* (*Rhizomucor miehei*)*, Thermomyces lanuginosus, Candida rugosa, Aspergillus oryzae, Pseudomonas cepacia, Pseudomonas fluorescens, Rhizopus delemar* et *Pseudomonas* sp. ainsi que leurs combinaisons, de préférence *Candida antarctica, Mucor miehei* (*Rhizomucor miehei*) et *Thermomyces lanuginosus;* et/ou
en particulier où l'enzyme est utilisée sous forme immobilisée, en particulier immobilisée sur un support, de préférence sur un support polymère, de préférence sur un support organique polymère, de manière particulièrement préférée avec des propriétés hydrophobes, de manière tout à fait préférée sur un support à base de résine poly(méth)acrylique; et/ou
en particulier où l'enzyme est recyclée après la réaction; et/ou
en particulier où la réaction est effectuée en présence d'une enzyme comme catalyseur à des températures dans la plage de 10 °C à 80 °C, en particulier dans la plage de 20 °C à 80 °C, de préférence dans la plage de 25 °C à 75 °C, de manière particulièrement préférée dans la plage de 45 °C à 75 °C, de manière tout particulièrement préférée dans la plage de 50 °C à 70 °C; et/ou
en particulier où l'enzyme est utilisée en quantités, par rapport à la quantité totale des composés de départ (I) et (II), dans la plage de 0,001 % en poids à 20 % en poids, en particulier dans la plage de 0,01 % en poids à 15 % en poids, de préférence dans la plage de 0,1 % en poids à 15 % en poids, de préférence dans la plage de 0,5 % en poids à 10 % en poids; et/ou
en particulier où la réaction est effectuée en présence d'une enzyme comme catalyseur à une pression dans la plage de 0,0001 bar à 10 bars, en particulier dans la plage de 0,001 bar à 5 bars, de préférence dans la plage de 0,01 bar à 2 bars, de manière particulièrement préférée dans la plage de 0,05 bar à 1 bar, tout particulièrement à environ 0,5 bar; et/ou
en particulier où la réaction est effectuée en présence d'une enzyme en présence d'un gaz inerte, en particulier en présence d'hélium, d'argon ou d'azote, de préférence en présence d'azote.

5. Procédé selon la revendication 1 ou la revendication 2,
où la réaction est effectuée en présence d'un catalyseur acide ou basique contenant un métal et/ou à base de métal;
en particulier où le catalyseur est choisi parmi (i) les catalyseurs basiques, en particulier les hydroxydes alcalins ou alcalino-terreux et les alcoolates alcalins ou alcalino-terreux, tels que NaOH, KOH, LiOH, Ca(OH)₂, NaOMe, KOMe et Na(OBu-tert.), (ii) des catalyseurs acides, en particulier des acides minéraux, et des acides organiques, tels que l'acide sulfurique, l'acide chlorhydrique, l'acide phosphorique, l'acide nitrique, les acides sulfoniques, l'acide méthanesulfonique, l'acide para-toluènesulfonique et les acides carboxyliques, (iii) des acides de Lewis, en particulier des acides de Lewis à base de composés de titane, d'étain, de zinc et d'aluminium, tels que le tétrabutylate de titane, les acides stanniques, l'acétate de zinc, le trichlorure d'aluminium et le triisopropyle d'aluminium, et (iv) des catalyseurs hétérogènes, notamment à base de silicates minéraux, de germanates, de carbonates et d'oxydes d'aluminium, tels que les zéolites, les montmorillonites, les mordénites, les hydrotalcites et les alumines, ainsi que leurs combinaisons; et/ou
en particulier où l'on utilise comme catalyseur un acide de Lewis à base de composés de titane, d'étain, de zinc et d'aluminium, comme le tétrabutylate de titane, les acides stanniques, l'acétate de zinc, le trichlorure d'aluminium et le triisopropyle d'aluminium; et/ou
en particulier où le catalyseur est recyclé après la réaction; et/ou
en particulier où la réaction est effectuée en présence d'un catalyseur acide ou basique contenant un métal et/ou à base de métal, à des températures dans la plage de 20 °C à 160 °C, en particulier dans la plage de 50 °C à 150 °C, de préférence dans la plage de 70 °C à 140 °C, de manière particulièrement préférée dans la plage de 80 °C à 135 °C, de manière tout à fait préférée dans la plage de 100 °C à 130 °C; et/ou
en particulier où le catalyseur est utilisé en quantités, par rapport à la quantité totale des composés de départ (I) et (II), dans la plage de 0,01 % en poids à 30 % en poids, en particulier dans la plage de 0,05 % en poids à 15 % en poids, de préférence dans la plage de 0,1 % en poids à 15 % en poids, de préférence dans la plage de 0,2 % en poids à 10 % en poids; et/ou
en particulier où la réaction est effectuée en présence d'un catalyseur acide ou basique contenant un métal et/ou à base de métal, à une pression dans la plage de 0,0001 bar à 10 bars, en particulier dans la plage de 0,001 bar à 5 bars, de préférence dans la plage de 0,01 bar à 2 bars, de manière particulièrement préférée dans la plage de 0,05 bar à 1 bar, tout particulièrement à environ 1 bar; et/ou
en particulier où la réaction est effectuée en présence d'un catalyseur acide ou basique contenant un métal et/ou à base de métal, en présence d'un gaz inerte, en particulier en présence d'hélium, d'argon ou d'azote, de préférence en présence d'azote.

6. Procédé selon l'une quelconque des revendications précédentes,
où l'oxobutanol de formule générale (I) est utilisé, par rapport aux groupes carboxyle de l'acide carboxylique polymère (II), en quantités molaires dans une plage allant d'une quantité équimolaire jusqu'à un excès molaire de 200 % en moles, en particulier dans une plage allant d'une quantité équimolaire jusqu'à un excès molaire de 150 % en moles, de préférence dans une plage allant d'une quantité équimolaire jusqu'à un excès molaire de 100 % en moles; et/ou
où l'oxobutanol de formule générale (I) et l'acide carboxylique polymère (II) sont utilisés dans un rapport molaire oxobutanol de formule générale (I)/groupes carboxyle de l'acide carboxylique polymère (II) dans une plage de 1:1 à 10:1, en particulier dans une plage de 2:1 à 8:1, de préférence dans une plage de 3:1 à 6:1.

7. Procédé selon l'une quelconque des revendications précédentes,
où les groupes hydroxyle et/ou les groupes carboxyle encore présents dans le produit de réaction après la réaction sont fonctionnalisés, en particulier estérifiés, au moins partiellement, de préférence complètement; et/ou
où la réaction est suivie d'une fonctionnalisation partielle, en particulier complète, en particulier d'une estérification, de groupes hydroxyle et/ou de groupes carboxyle encore présents.

8. Produit de réaction pouvant être obtenu par le procédé selon l'une quelconque des revendications précédentes.

9. Esters d'oxobutanol d'un acide carboxylique polymère,
où l'acide carboxylique polymère est choisi dans le groupe constitué par l'acide polytartrique, l'acide polymalique et l'acide polycitrique ainsi que leurs sels et sels partiels et esters et esters partiels et leurs combinaisons et mélanges; et
où au moins un groupe carboxyle, en particulier au moins un groupe carboxyle terminal et/ou primaire, de l'acide carboxylique polymère est estérifié avec un oxobutanol de formule générale (I)
CH₃ - CH(OH) - CH₂ - C(O)OR¹ (I)
où, dans la formule générale (I), le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle.

10. Ester d'oxobutanol d'un acide carboxylique polymère selon la revendication 9,
où l'acide carboxylique polymère correspond à la formule générale (IId) 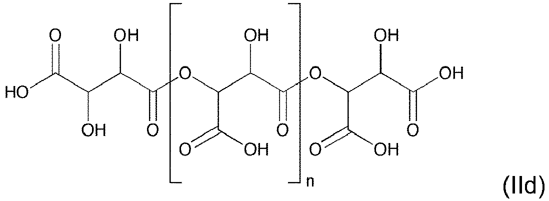
où, dans la formule générale (IId), la variable n représente un nombre (entier) ≥ 2, en particulier dans la plage de deux à vingt, de préférence dans la plage de deux à dix, de manière particulièrement préférée dans la plage de trois à sept.

11. Ester d'oxobutanol d'un acide carboxylique polymère selon la revendication 9 ou la revendication 10,
où l'ester d'oxobutanol correspond à la formule générale (IIId) 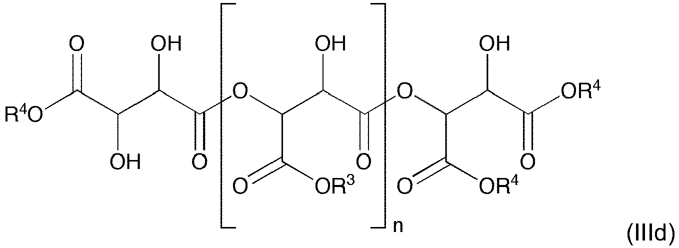
où, dans la formule générale (IIId)
• la variable n représente un nombre (entier) ≥ 2, en particulier dans la plage de deux à vingt, de préférence dans la plage de deux à dix, de manière particulièrement préférée dans la plage de trois à sept;
• R³, indépendamment l'un de l'autre par rapport à l'unité de répétition respective représente: l'hydrogène ou un radical - CH(CH₃ ) - CH₂ - C(O)OR¹ où le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle;
• R⁴, indépendamment l'un de l'autre, représente l'hydrogène ou un radical - CH(CH₃) - CH₂ - C(O)OR¹, où le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle;
à condition toutefois qu'au moins un, en particulier au moins deux, des radicaux R³ et R⁴ représente un radical - CH(CH₃) - CH₂ - C(O)OR¹, tel que défini précédemment, de préférence à la condition (supplémentaire) qu'au moins un radical R⁴ représente un radical - CH(CH₃) - CH₂ - C(O)OR¹, tel que défini précédemment.

12. Mélange comprenant au moins deux, en particulier au moins trois, esters d'oxobutanol différents les uns des autres d'un acide carboxylique polymère, tel que défini dans les revendications 8 à 11 précédentes.

13. Composition pharmaceutique, en particulier un médicament ou un médicament, comprenant un produit de réaction selon la revendication 8 et/ou un ester d'oxobutanol d'un acide carboxylique polymère selon l'une quelconque des revendications 9 à 11 et/ou un mélange selon la revendication 12.

14. Composition pharmaceutique selon la revendication 13 pour l'utilisation dans le traitement prophylactique et/ou thérapeutique de maladies du corps humain ou animal, en particulier de maladies liées à un trouble du métabolisme énergétique, en particulier du métabolisme des corps cétoniques, comme notamment les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

15. Produit de réaction selon la revendication 8 et/ou ester d'oxobutanol d'un acide carboxylique polymère selon l'une quelconque des revendications 9 à 11 et/ou mélange selon la revendication 12, destiné à être utilisé dans le traitement prophylactique et/ou thérapeutique de pathologies du corps humain ou animal, notamment les maladies liées à une perturbation du métabolisme énergétique, en particulier du métabolisme des corps cétoniques, telles que notamment les traumatismes crâniens, les accidents vasculaires cérébraux, les hypoxies, les maladies cardiovasculaires telles que l'infarctus du myocarde, le syndrome de réalimentation, les anorexies, l'épilepsie, les maladies neurodégénératives telles que la démence, la maladie d'Alzheimer, la maladie de Parkinson, sclérose en plaques et sclérose latérale amyotrophique, maladies du métabolisme lipidique telles que le déficit en transporteur de glucose (déficit GLUT1), VL-FAOD et mitochondriopathies telles que le déficit en thiolase mitochondriale, chorée de Huntington, maladies cancéreuses telles que lymphomes à cellules T, astrocytomes et glioblastomes, VIH, maladies rhumatismales telles que polyarthrite rhumatoïde et arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

16. Utilisation d'un produit de réaction selon la revendication 8 et/ou d'un ester d'oxobutanol d'un acide carboxylique polymère selon l'une des revendications 9 à 11 et/ou d'un mélange selon la revendication 12 pour la préparation d'un médicament destiné au traitement prophylactique et/ou thérapeutique de maladies du corps humain ou animal, notamment les maladies liées à une perturbation du métabolisme énergétique, en particulier du métabolisme des corps cétoniques, telles que notamment les traumatismes crâniens, les accidents vasculaires cérébraux, les hypoxies, les maladies cardiovasculaires telles que l'infarctus du myocarde, le syndrome de réalimentation, les anorexies, l'épilepsie, les maladies neurodégénératives telles que la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, en particulier la colite ulcéreuse et la maladie de Crohn, les maladies de stockage lyosomal telles que les sphingolipidoses, en particulier la maladie de Niemann-Pick, le diabète sucré et les effets ou effets secondaires des chimiothérapies.

17. Utilisation d'un produit de réaction selon la revendication 8 et/ou d'un ester d'oxobutanol d'un acide carboxylique polymère selon l'une quelconque des revendications 9 à 11 et/ou d'un mélange selon la revendication 12 pour la préparation d'un médicament destiné au traitement prophylactique et/ou thérapeutique ou à l'application de/à des situations métaboliques cataboliques, telles que la faim, les régimes ou les régimes pauvres en hydrates de carbone.

18. Produit alimentaire et/ou nutritionnel comprenant un produit de réaction selon la revendication 8 et/ou un ester d'oxobutanol d'un acide carboxylique polymère selon l'une quelconque des revendications 9 à 11 et/ou un mélange selon la revendication 12.
